# EUROPÄISCHE PATENTSCHRIFT

(11) **EP 2 421 960 B1**
(45) Veröffentlichungstag und Bekanntmachung des Hinweises auf die Patenterteilung: **19.07.2017**
(21) Anmeldenummer: 10704577.5
(22) Anmeldetag: 23.02.2010
(51) Int. Cl.: C12N 9/10, C12N 9/16, C12N 9/88, C12P 7/28, C12R 1/145

(54) **ZELLEN UND VERFAHREN ZUR HERSTELLUNG VON ACETON**
CELLS AND METHODS FOR PRODUCING ACETONE
CELLULES ET MÉTHODES POUR LA PRODUCTION D'ACÉTONE

(30) Priorität: 23.04.2009 DE 102009002583
(43) Veröffentlichungstag der Anmeldung: 29.02.2012
(73) Patentinhaber: Evonik Degussa GmbH, 45128 Essen (DE)
(72) Erfinder: BECKER, Ulrich, 63579 Freigericht-Horbach (DE); GRUND, Gerda, 48653 Coesfeld (DE); ORSCHEL, Matthias, 48149 Münster (DE); DODERER, Kai, 63110 Rodgau (DE); LÖHDEN, Gerd, 45136 Essen (DE); BRAND, Gerd, 48301 Nottuln/Schapdetten (DE); DÜRRE, Peter, 89075 Ulm (DE); THUM, Simone, 89077 Ulm (DE); BAHL, Hubert Johannes, 18059 Rostock (DE); FISCHER, Ralf-Jörg, 18198 Kritzmow (DE); MAY, Antje, 18057 Rostock (DE)
(86) Internationale Anmeldenummer: PCT/EP2010/052244
(87) Internationale Veröffentlichungsnummer: WO 2010/121849

(56) Entgegenhaltungen:
- WO-A2-2009/056423
- MERMELSTEIN LEE D ET AL: "Metabolic engineering of Clostridium acetobutylicum ATCC 824 for increased solvent production by enhancement of acetone formation enzyme activities using a synthetic acetone operon" BIOTECHNOLOGY AND BIOENGINEERING, Bd. 42, Nr. 9, 1993, Seiten 1053-1060, XP002579042 ISSN: 0006-3592
- MOLLAH A H ET AL: "THE INFLUENCE OF HYDROGEN CARBON DIOXIDE AND DILUTION RATE ON THE CONTINUOUS FERMENTATION OF ACETONE BUTANOL" APPLIED MICROBIOLOGY AND BIOTECHNOLOGY, SPRINGER VERLAG, BERLIN, DE, Bd. 37, Nr. 5, 1. Januar 1992 (1992-01-01) , Seiten 533-538, XP009132534 ISSN: 0175-7598
- KIM B H ET AL: "CONTROL OF CARBON AND ELECTRON FLOW IN CLOSTRIDIUM-ACETOBUTYLICUM FERMENTATIONS UTILIZATION OF CARBON MONOXIDE TO INHIBIT HYDROGEN PRODUCTION AND TO ENHANCE BUTANOL YIELDS" APPLIED AND ENVIRONMENTAL MICROBIOLOGY, Bd. 48, Nr. 4, 1984, Seiten 764-770, XP002579043 ISSN: 0099-2240
- Hiroki Matsui ET AL: "Diversity of the Formyltetrahydrofolate Synthetase Gene ( fhs ), a Key Enzyme for Reductive Acetogenesis, in the Bovine Rumen", Bioscience, Biotechnology, and Biochemistry, vol. 72, no. 12, 23 December 2008 (2008-12-23), pages 3273-3276, XP055158612, ISSN: 0916-8451, DOI: 10.1271/bbb.70375
- BERMEJO L L ET AL: "Expression of Clostridium acetobutylicum ATCC 824 genes in Escherichia coli for acetone production and acetate detoxification", APPLIED AND ENVIRONMENTAL MICROBIOLOGY, AMERICAN SOCIETY FOR MICROBIOLOGY, US, vol. 64, no. 3, 1 March 1998 (1998-03-01), pages 1079-1085, XP002334443, ISSN: 0099-2240
- HENSTRA ET AL: "Microbiology of synthesis gas fermentation for biofuel production", CURRENT OPINION IN BIOTECHNOLOGY, LONDON, GB, vol. 18, no. 3, 8 June 2007 (2007-06-08), pages 200-206, XP022110181, ISSN: 0958-1669, DOI: 10.1016/J.COPBIO.2007.03.008

### SEQUENCE LISTING

<110> Evonik Degussa
<120> Zellen und Verfahren zur Herstellung von Aceton
<130> 200900033
<160> 16
<170> PatentIn version 3.4
<210> 1
   <211> 17
   <212> DNA
   <213> Artificial
<220>
   <223> Primer
<400> 1
   ggaaggtacc ttttatg 17
<210> 2
   <211> 25
   <212> DNA
   <213> Artificial
<220>
   <223> Primer
<400> 2
   gtaactctga attctattac ttaag 25
<210> 3
   <211> 20
   <212> DNA
   <213> Artificial
<220>
   <223> Primer
<400> 3
   cacaacggtg gatccaagag 20
<210> 4
   <211> 20
   <212> DNA
   <213> Artificial
<220>
   <223> Primer
<400> 4
   cgcgatatgg taccaatcat 20
<210> 5
   <211> 28
   <212> DNA
   <213> Artificial
<220>
   <223> Primer
<400> 5
   gaatttaaaa ggagggatcc aaatgaac 28
<210> 6
   <211> 27
   <212> DNA
   <213> Artificial
<220>
   <223> Primer
<400> 6
   gtttcatagt attggtacct aaacagc 27
<210> 7
   <211> 20
   <212> DNA
   <213> Artificial
<220>
   <223> Primer
<400> 7
   ctcaggtcga cttcaagaag 20
<210> 8
   <211> 29
   <212> DNA
   <213> Artificial
<220>
   <223> Primer
<400> 8
   cagagttatt tttaaggatc ctttctagc 29
<210> 9
   <211> 30
   <212> DNA
   <213> Artificial
<220>
   <223> Primer
<400> 9
   caattgggat ccgataacaa tttcacacag 30
<210> 10
   <211> 29
   <212> DNA
   <213> Artificial
<220>
   <223> Primer
<400> 10
   gagatctggt acccggttaa atgatcgga 29
<210> 11
   <211> 28
   <212> DNA
   <213> Artificial
<220>
   <223> Primer
<400> 11
   ctctagaagg atcctgttta actttaag 28
<210> 12
   <211> 25
   <212> DNA
   <213> Artificial
<220>
   <223> Primer
<400> 12
   attgggtacc tcattgcata ctccg 25
<210> 13
   <211> 8559
   <212> DNA
   <213> Artificial
<220>
   <223> Plasmid
<400> 13
<210> 14
   <211> 8563
   <212> DNA
   <213> Artificial
<220>
   <223> Plasmid
<400> 14
   agcttggctg caggtcgact tcaagaagag gcacctcatc ttggaaagcc tgtacttgtt 60
   ttaagagatg ttactgaaag accagaagca gttgaggcag gaacggtaaa attagtagga 120
   actgatatta aaaagatagt agacgaagcc tataaaataa tgaaagatga agaagaatat 180
   gaaaaaatga gtaaggcaat aaatccatat ggtgatggaa aggcttcaga tagaatagtt 240
   gatgctatat tgtatcattt tggagtatta aaggatagac cagatgaatt ttcacctaaa 300
   aagtaataca tgagcattct aaaagaattt agaatgaagt ttcttatgca caagtatttt 360
   ttattacatt aatatagtta aaatataaac ttatgtattt atgctaaaac atgattttaa 420
   gggggttagc atatgcataa gtttaatttt tttgttaaaa aatattaaac tttgtgtttt 480
   ttttaacaaa atatattgat aaaaataata atagtgggta taattaagtt gttagagaaa 540
   acgtataaat tagggataaa ctatggaact tatgaaatag attgaaatgg tttatctgtt 600
   accccgtatc aaaatttagg aggttagtta gaatgaaaga agttgtaata gctagtgcag 660
   taagaacagc gattggatct tatggaaagt ctcttaagga tgtaccagca gtagatttag 720
   gagctacagc tataaaggaa gcagttaaaa aagcaggaat aaaaccagag gatgttaatg 780
   aagtcatttt aggaaatgtt cttcaagcag gtttaggaca gaatccagca agacaggcat 840
   cttttaaagc aggattacca gttgaaattc cagctatgac tattaataag gtttgtggtt 900
   caggacttag aacagttagc ttagcagcac aaattataaa agcaggagat gctgacgtaa 960
   taatagcagg tggtatggaa aatatgtcta gagctcctta cttagcgaat aacgctagat 1020
   ggggatatag aatgggaaac gctaaatttg ttgatgaaat gatcactgac ggattgtggg 1080
   atgcatttaa tgattaccac atgggaataa cagcagaaaa catagctgag agatggaaca 1140
   tttcaagaga agaacaagat gagtttgctc ttgcatcaca aaaaaaagct gaagaagcta 1200
   taaaatcagg tcaatttaaa gatgaaatag ttcctgtagt aattaaaggc agaaagggag 1260
   aaactgtagt tgatacagat gagcacccta gatttggatc aactatagaa ggacttgcaa 1320
   aattaaaacc tgccttcaaa aaagatggaa cagttacagc tggtaatgca tcaggattaa 1380
   atgactgtgc agcagtactt gtaatcatga gtgcagaaaa agctaaagag cttggagtaa 1440
   aaccacttgc taagatagtt tcttatggtt cagcaggagt tgacccagca ataatgggat 1500
   atggaccttt ctatgcaaca aaagcagcta ttgaaaaagc aggttggaca gttgatgaat 1560
   tagatttaat agaatcaaat gaagcttttg cagctcaaag tttagcagta gcaaaagatt 1620
   taaaatttga tatgaataaa gtaaatgtaa atggaggagc tattgccctt ggtcatccaa 1680
   ttggagcatc aggtgcaaga atactcgtta ctcttgtaca cgcaatgcaa aaaagagatg 1740
   caaaaaaagg cttagcaact ttatgtatag gtggcggaca aggaacagca atattgctag 1800
   aaaagtgcta gaaaggatcc aagagggata aaaaatgaaa acaaaattga tgacattaca 1860
   agacgccacc ggcttctttc gtgacggcat gaccatcatg gtgggcggat ttatggggat 1920
   tggcactcca tcccgcctgg ttgaagcatt actggaatct ggtgttcgcg acctgacatt 1980
   gatagccaat gataccgcgt ttgttgatac cggcatcggt ccgctcatcg tcaatggtcg 2040
   agtccgcaaa gtgattgctt cacatatcgg caccaacccg gaaacaggtc ggcgcatgat 2100
   atctggtgag atggacgtcg ttctggtgcc gcaaggtacg ctaatcgagc aaattcgctg 2160
   tggtggagct ggacttggtg gttttctcac cccaacgggt gtcggcaccg tcgtagagga 2220
   aggcaaacag acactgacac tcgacggtaa aacctggctg ctcgaacgcc cactgcgcgc 2280
   cgacctggcg ctaattcgcg ctcatcgttg cgacacactt ggcaacctga cctatcaact 2340
   tagcgcccgc aactttaacc ccctgatagc ccttgcggct gatatcacgc tggtagagcc 2400
   agatgaactg gtcgaaaccg gcgagctgca acctgaccat attgtcaccc ctggtgccgt 2460
   tatcgaccac atcatcgttt cacaggagag caaataatgg atgcgaaaca acgtattgcg 2520
   cgccgtgtgg cgcaagagct tcgtgatggt gacatcgtta acttagggat cggtttaccc 2580
   acaatggtcg ccaattattt accggagggt attcatatca ctctgcaatc ggaaaacggc 2640
   ttcctcggtt taggcccggt cacgacagcg catccagatc tggtgaacgc tggcgggcaa 2700
   ccgtgcggtg ttttacccgg tgcagccatg tttgatagcg ccatgtcatt tgcgctaatc 2760
   cgtggcggtc atattgatgc ctgcgtgctc ggcggtttgc aagtagacga agaagcaaac 2820
   ctcgcgaact gggtagtgcc tgggaaaatg gtgcccggta tgggtggcgc gatggatctg 2880
   gtgaccgggt cgcgcaaagt gatcatcgcc atggaacatt gcgccaaaga tggttcagca   2940
   aaaattttgc gccgctgcac catgccactc actgcgcaac atgcggtgca tatgctggtt   3000
   actgaactgg ctgtctttcg ttttattgac ggcaaaatgt ggctcaccga aattgccgac   3060
   gggtgtgatt tagccaccgt gcgtgccaaa acagaagctc ggtttgaagt cgccgccgat   3120
   ctgaatacgc aacggggtga tttatgattg gtacctttta tgttaaagga tgaagtaatt   3180
   aaacaaatta gcacgccatt aacttcgcct gcatttccta gaggacccta taaatttcat   3240
   aatcgtgagt attttaacat tgtatatcgt acagatatgg atgcacttcg taaagttgtg   3300
   ccagagcctt tagaaattga tgagccctta gtcaggtttg aaattatggc aatgcatgat   3360
   acgagtggac ttggttgtta tacagaaagc ggacaggcta ttcccgtaag ctttaatgga   3420
   gttaagggag attatcttca tatgatgtat ttagataatg agcctgcaat tgcagtagga   3480
   agggaattaa gtgcatatcc taaaaagctc gggtatccaa agctttttgt ggattcagat   3540
   actttagtag gaactttaga ctatggaaaa cttagagttg cgacagctac aatggggtac   3600
   aaacataaag ccttagatgc taatgaagca aaggatcaaa tttgtcgccc taattatatg   3660
   ttgaaaataa tacccaatta tgatggaagc cctagaatat gtgagcttat aaatgcgaaa   3720
   atcacagatg ttaccgtaca tgaagcttgg acaggaccaa ctcgactgca gttatttgat   3780
   cacgctatgg cgccacttaa tgatttgcca gtaaaagaga ttgtttctag ctctcacatt   3840
   cttgcagata taatattgcc tagagctgaa gttatatatg attatcttaa gtaatagaat   3900
   tcactggccg tcgttttaca acgtcgtgac tgggaaaacc ctggcgttac ccaacttaat   3960
   cgccttgcag cacatccccc tttcgccagc tggcgtaata gcgaagaggc ccgcaccgat   4020
   cgcccttccc aacagttgcg cagcctgaat ggcgaatggc gcctgatgcg gtattttctc   4080
   cttacgcatc tgtgcggtat ttcacaccgc atatggtgca ctctcagtac aatctgctct   4140
   gatgccgcat agttaagcca gccccgacac ccgccaacac ccgctgacgc gccctgacgg   4200
   gcttgtctgc tcccggcatc cgcttacaga caagctgtga ccgtctccgg gagctgcatg   4260
   tgtcagaggt tttcaccgtc atcaccgaaa cgcgcgagac gaaagggcct cgtgatacgc   4320
   ctatttttat aggttaatgt catgataata atggtttctt agacgtcagg tggcactttt   4380
   cggggaaatg tgcgcggaac ccctatttgt ttatttttct aaatacattc aaatatgtat   4440
   ccgctcatga gacaataacc ctgataaatg cttcaataat attgaaaaag gaagagtatg   4500
   agtattcaac atttccgtgt cgcccttatt cccttttttg cggcattttg ccttcctgtt   4560
   tttgctcacc cagaaacgct ggtgaaagta aaagatgctg aagatcagtt gggtgcacga   4620
   gtgggttaca tcgaactgga tctcaacagc ggtaagatcc ttgagagttt tcgccccgaa   4680
   gaacgttttc caatgatgag cacttttaaa gttctgctat gtggcgcggt attatcccgt   4740
   attgacgccg ggcaagagca actcggtcgc cgcatacact attctcagaa tgacttggtt   4800
   gagtactcac cagtcacaga aaagcatctt acggatggca tgacagtaag agaattatgc   4860
   agtgctgcca taaccatgag tgataacact gcggccaact tacttctgac aacgatcgga   4920
   ggaccgaagg agctaaccgc ttttttgcac aacatggggg atcatgtaac tcgccttgat   4980
   cgttgggaac cggagctgaa tgaagccata ccaaacgacg agcgtgacac cacgatgcct   5040
   gtagcaatgg caacaacgtt gcgcaaacta ttaactggcg aactacttac tctagcttcc   5100
   cggcaacaat taatagactg gatggaggcg gataaagttg caggaccact tctgcgctcg   5160
   gcccttccgg ctggctggtt tattgctgat aaatctggag ccggtgagcg tgggtctcgc   5220
   ggtatcattg cagcactggg gccagatggt aagccctccc gtatcgtagt tatctacacg   5280
   acggggagtc aggcaactat ggatgaacga aatagacaga tcgctgagat aggtgcctca   5340
   ctgattaagc attggtaact gtcagaccaa gtttactcat atatacttta gattgattta   5400
   aaacttcatt tttaatttaa aaggatctag gtgaagatcc tttttgataa tctcatgacc   5460
   aaaatccctt aacgtgagtt ttcgttccac tgagcgtcag accccgtaga aaagatcaaa   5520
   ggatcttctt gagatccttt ttttctgcgc gtaatctgct gcttgcaaac aaaaaaacca   5580
   ccgctaccag cggtggtttg tttgccggat caagagctac caactctttt tccgaaggta   5640
   actggcttca gcagagcgca gataccaaat actgtccttc tagtgtagcc gtagttaggc   5700
   caccacttca agaactctgt agcaccgcct acatacctcg ctctgctaat cctgttacca   5760
   gtggctgctg ccagtggcga taagtcgtgt cttaccgggt tggactcaag acgatagtta   5820
   ccggataagg cgcagcggtc gggctgaacg gggggttcgt gcacacagcc cagcttggag   5880
   cgaacgacct acaccgaact gagataccta cagcgtgagc tatgagaaag cgccacgctt   5940
   cccgaaggga gaaaggcgga caggtatccg gtaagcggca gggtcggaac aggagagcgc   6000
   acgagggagc ttccaggggg aaacgcctgg tatctttata gtcctgtcgg gtttcgccac   6060
   ctctgacttg agcgtcgatt tttgtgatgc tcgtcagggg ggcggagcct atggaaaaac   6120
   gccagcaacg cggccttttt acggttcctg gccttttgct ggccttttgc tcacatgttc   6180
   tttcctgcgt tatcccctga ttctgtggat aaccgtatta ccgcctttga gtgagctgat   6240
   accgctcgcc gcagccgaac gaccgagcgc agcgagtcag tgagcgagga agcggaagag   6300
   cgcccaatac gcaaaccgcc tctccccgcg cgttggccga ttcattaatg cagctggcac   6360
   gacaggtttc ccgactggaa agcgggcagt gagcgcaacg caattaatgt gagttagctc   6420
   actcattagg caccccaggc tttacacttt atgcttccgg ctcgtatgtt gtgtggaatt   6480
   gtgagcggat aacaatttca cacaggaaac agctatgacc atgattacgc caagctttgg   6540
   ctaacacaca cgccattcca accaatagtt ttctcggcat aaagccatgc tctgacgctt   6600
   aaatgcacta atgccttaaa aaaacattaa agtctaacac actagactta tttacttcgt   6660
   aattaagtcg ttaaaccgtg tgctctacga ccaaaagtat aaaaccttta agaactttct   6720
   tttttcttgt aaaaaaagaa actagataaa tctctcatat cttttattca ataatcgcat   6780
   cagattgcag tataaattta acgatcactc atcatgttca tatttatcag agctcgtgct   6840
   ataattatac taattttata aggaggaaaa aataaagagg gttataatga acgagaaaaa   6900
   tataaaacac agtcaaaact ttattacttc aaaacataat atagataaaa taatgacaaa   6960
   tataagatta aatgaacatg ataatatctt tgaaatcggc tcaggaaaag ggcattttac   7020
   ccttgaatta gtacagaggt gtaatttcgt aactgccatt gaaatagacc ataaattatg   7080
   caaaactaca gaaaataaac ttgttgatca cgataatttc caagttttaa acaaggatat   7140
   attgcagttt aaatttccta aaaaccaatc ctataaaata tttggtaata taccttataa   7200
   cataagtacg gatataatac gcaaaattgt ttttgatagt atagctgatg agatttattt   7260
   aatcgtggaa tacgggtttg ctaaaagatt attaaataca aaacgctcat tggcattatt   7320
   tttaatggca gaagttgata tttctatatt aagtatggtt ccaagagaat attttcatcc   7380
   taaacctaaa gtgaatagct cacttatcag attaaataga aaaaaatcaa gaatatcaca   7440
   caaagataaa cagaagtata attatttcgt tatgaaatgg gttaacaaag aatacaagaa   7500
   aatatttaca aaaaatcaat ttaacaattc cttaaaacat gcaggaattg acgatttaaa   7560
   caatattagc tttgaacaat tcttatctct tttcaatagc tataaattat ttaataagta   7620
   agttaaggga tgcataaact gcatccctta acttgttttt cgtgtaccta ttttttgtga   7680
   atcgattatg tcttttgcgc attcacttct tttctatata aatatgagcg aagcgaataa   7740
   gcgtcggaaa agcagcaaaa agtttccttt ttgctgttgg agcatggggg ttcagggggt   7800
   gcagtatctg acgtcaatgc cgagcgaaag cgagccgaag ggtagcattt acgttagata   7860
   accccctgat atgctccgac gctttatata gaaaagaaga ttcaactagg taaaatctta   7920
   atataggttg agatgataag gtttataagg aatttgtttg ttctaatttt tcactcattt   7980
   tgttctaatt tcttttaaca aatgttcttt tttttttaga acagttatga tatagttaga   8040
   atagtttaaa ataaggagtg agaaaaagat gaaagaaaga tatggaacag tctataaagg   8100
   ctctcagagg ctcatagacg aagaaagtgg agaagtcata gaggtagaca agttataccg   8160
   taaacaaacg tctggtaact tcgtaaaggc atatatagtg caattaataa gtatgttaga   8220
   tatgattggc ggaaaaaaac ttaaaatcgt taactatatc ctagataatg tccacttaag   8280
   taacaataca atgatagcta caacaagaga aatagcaaaa gctacaggaa caagtctaca   8340
   aacagtaata acaacactta aaatcttaga agaaggaaat attataaaaa gaaaaactgg   8400
   agtattaatg ttaaaccctg aactactaat gagaggcgac gaccaaaaac aaaaatacct   8460
   cttactcgaa tttgggaact ttgagcaaga ggcaaatgaa atagattgac ctcccaataa   8520
   caccacgtag ttattgggag gtcaatctat gaaaatgcga tta                     8563
<210> 15
   <211> 8004
   <212> DNA
   <213> Artificial
<220>
   <223> Plasmid
<400> 15
   agcttggctg caggtcgact tcaagaagag gcacctcatc ttggaaagcc tgtacttgtt     60
   ttaagagatg ttactgaaag accagaagca gttgaggcag gaacggtaaa attagtagga    120
   actgatatta aaaagatagt agacgaagcc tataaaataa tgaaagatga agaagaatat    180
   gaaaaaatga gtaaggcaat aaatccatat ggtgatggaa aggcttcaga tagaatagtt    240
   gatgctatat tgtatcattt tggagtatta aaggatagac cagatgaatt ttcacctaaa    300
   aagtaataca tgagcattct aaaagaattt agaatgaagt ttcttatgca caagtatttt    360
   ttattacatt aatatagtta aaatataaac ttatgtattt atgctaaaac atgattttaa    420
   gggggttagc atatgcataa gtttaatttt tttgttaaaa aatattaaac tttgtgtttt    480
   ttttaacaaa atatattgat aaaaataata atagtgggta taattaagtt gttagagaaa    540
   acgtataaat tagggataaa ctatggaact tatgaaatag attgaaatgg tttatctgtt    600
   accccgtatc aaaatttagg aggttagtta gaatgaaaga agttgtaata gctagtgcag    660
   taagaacagc gattggatct tatggaaagt ctcttaagga tgtaccagca gtagatttag    720
   gagctacagc tataaaggaa gcagttaaaa aagcaggaat aaaaccagag gatgttaatg    780
   aagtcatttt aggaaatgtt cttcaagcag gtttaggaca gaatccagca agacaggcat    840
   cttttaaagc aggattacca gttgaaattc cagctatgac tattaataag gtttgtggtt    900
   caggacttag aacagttagc ttagcagcac aaattataaa agcaggagat gctgacgtaa    960
   taatagcagg tggtatggaa aatatgtcta gagctcctta cttagcgaat aacgctagat   1020
   ggggatatag aatgggaaac gctaaatttg ttgatgaaat gatcactgac ggattgtggg   1080
   atgcatttaa tgattaccac atgggaataa cagcagaaaa catagctgag agatggaaca   1140
   tttcaagaga agaacaagat gagtttgctc ttgcatcaca aaaaaaagct gaagaagcta   1200
   taaaatcagg tcaatttaaa gatgaaatag ttcctgtagt aattaaaggc agaaagggag   1260
   aaactgtagt tgatacagat gagcacccta gatttggatc aactatagaa ggacttgcaa   1320
   aattaaaacc tgccttcaaa aaagatggaa cagttacagc tggtaatgca tcaggattaa   1380
   atgactgtgc agcagtactt gtaatcatga gtgcagaaaa agctaaagag cttggagtaa   1440
   aaccacttgc taagatagtt tcttatggtt cagcaggagt tgacccagca ataatgggat   1500
   atggaccttt ctatgcaaca aaagcagcta ttgaaaaagc aggttggaca gttgatgaat   1560
   tagatttaat agaatcaaat gaagcttttg cagctcaaag tttagcagta gcaaaagatt   1620
   taaaatttga tatgaataaa gtaaatgtaa atggaggagc tattgccctt ggtcatccaa   1680
   ttggagcatc aggtgcaaga atactcgtta ctcttgtaca cgcaatgcaa aaaagagatg   1740
   caaaaaaagg cttagcaact ttatgtatag gtggcggaca aggaacagca atattgctag   1800
   aaaagtgcta gaaaggatcc gataacaatt tcacacagaa ttcattaaag aggagaaatt   1860
   aaccatgggc caactcttca aatcatttga tgcgtcggaa aaaacaccgc tcatctgttt   1920
   tccgtttgcc ggcggctatt cggcgtcgtt tcgccctctc catgcttttt tgcaggggga   1980
   gtgcgagatg ctcgctgccg agccgccggg acacggcacg aatcaaacgt cagccattga   2040
   ggatctcgaa gagctgacgg atttgtacaa gcaagaactg aaccttcgcc ctgatcggcc   2100
   gtttgtgctg ttcggacaca gtatgggcgg aatgatcacc ttcaggctgg cgcaaaagct 2160
   tgagcgtgaa ggcatctttc cgcaggcggt tatcatttct gcaatccagc cgcctcatat 2220
   tcagcggagg aaagtgtccc acctgcctga tgatcagttt ctcgatcata ttatccaatt 2280
   aggcggaatg cccgcagagc ttgttgaaaa taaggaggtc atgtcctttt tcctgccttc 2340
   tttccgatca gattaccggg ctcttgaaca atttgagctt tacgatctgg cccagatcca 2400
   gtcgcctgtt catgtcttta acgggcttga tgataaaaaa tgcatacgag atgcggaagg 2460
   gtggaagaag tgggcaaaag acatcacatt ccatcaattt gacggcgggc acatgttcct 2520
   gctgtcacaa acggaagaag tcgcagaacg gatttttgcg atcttgaatc agcatccgat 2580
   catttaaccg ggtacctttt atgttaaagg atgaagtaat taaacaaatt agcacgccat 2640
   taacttcgcc tgcatttcct agaggaccct ataaatttca taatcgtgag tattttaaca 2700
   ttgtatatcg tacagatatg gatgcacttc gtaaagttgt gccagagcct ttagaaattg 2760
   atgagccctt agtcaggttt gaaattatgg caatgcatga tacgagtgga cttggttgtt 2820
   atacagaaag cggacaggct attcccgtaa gctttaatgg agttaaggga gattatcttc 2880
   atatgatgta tttagataat gagcctgcaa ttgcagtagg aagggaatta agtgcatatc 2940
   ctaaaaagct cgggtatcca aagctttttg tggattcaga tactttagta ggaactttag 3000
   actatggaaa acttagagtt gcgacagcta caatggggta caaacataaa gccttagatg 3060
   ctaatgaagc aaaggatcaa atttgtcgcc ctaattatat gttgaaaata atacccaatt 3120
   atgatggaag ccctagaata tgtgagctta taaatgcgaa aatcacagat gttaccgtac 3180
   atgaagcttg gacaggacca actcgactgc agttatttga tcacgctatg gcgccactta 3240
   atgatttgcc agtaaaagag attgtttcta gctctcacat tcttgcagat ataatattgc 3300
   ctagagctga agttatatat gattatctta agtaatagaa ttcactggcc gtcgttttac 3360
   aacgtcgtga ctgggaaaac cctggcgtta cccaacttaa tcgccttgca gcacatcccc 3420
   ctttcgccag ctggcgtaat agcgaagagg cccgcaccga tcgcccttcc caacagttgc 3480
   gcagcctgaa tggcgaatgg cgcctgatgc ggtattttct ccttacgcat ctgtgcggta 3540
   tttcacaccg catatggtgc actctcagta caatctgctc tgatgccgca tagttaagcc 3600
   agccccgaca cccgccaaca cccgctgacg cgccctgacg ggcttgtctg ctcccggcat 3660
   ccgcttacag acaagctgtg accgtctccg ggagctgcat gtgtcagagg ttttcaccgt 3720
   catcaccgaa acgcgcgaga cgaaagggcc tcgtgatacg cctattttta taggttaatg 3780
   tcatgataat aatggtttct tagacgtcag gtggcacttt tcggggaaat gtgcgcggaa 3840
   cccctatttg tttatttttc taaatacatt caaatatgta tccgctcatg agacaataac 3900
   cctgataaat gcttcaataa tattgaaaaa ggaagagtat gagtattcaa catttccgtg 3960
   tcgcccttat tccctttttt gcggcatttt gccttcctgt ttttgctcac ccagaaacgc 4020
   tggtgaaagt aaaagatgct gaagatcagt tgggtgcacg agtgggttac atcgaactgg 4080
   atctcaacag cggtaagatc cttgagagtt ttcgccccga agaacgtttt ccaatgatga 4140
   gcacttttaa agttctgcta tgtggcgcgg tattatcccg tattgacgcc gggcaagagc 4200
   aactcggtcg ccgcatacac tattctcaga atgacttggt tgagtactca ccagtcacag 4260
   aaaagcatct tacggatggc atgacagtaa gagaattatg cagtgctgcc ataaccatga 4320
   gtgataacac tgcggccaac ttacttctga caacgatcgg aggaccgaag gagctaaccg 4380
   cttttttgca caacatgggg gatcatgtaa ctcgccttga tcgttgggaa ccggagctga 4440
   atgaagccat accaaacgac gagcgtgaca ccacgatgcc tgtagcaatg gcaacaacgt 4500
   tgcgcaaact attaactggc gaactactta ctctagcttc ccggcaacaa ttaatagact 4560
   ggatggaggc ggataaagtt gcaggaccac ttctgcgctc ggcccttccg gctggctggt 4620
   ttattgctga taaatctgga gccggtgagc gtgggtctcg cggtatcatt gcagcactgg 4680
   ggccagatgg taagccctcc cgtatcgtag ttatctacac gacggggagt caggcaacta 4740
   tggatgaacg aaatagacag atcgctgaga taggtgcctc actgattaag cattggtaac 4800
   tgtcagacca agtttactca tatatacttt agattgattt aaaacttcat ttttaattta 4860
   aaaggatcta ggtgaagatc ctttttgata atctcatgac caaaatccct taacgtgagt 4920
   tttcgttcca ctgagcgtca gaccccgtag aaaagatcaa aggatcttct tgagatcctt 4980
   tttttctgcg cgtaatctgc tgcttgcaaa caaaaaaacc accgctacca gcggtggttt 5040
   gtttgccgga tcaagagcta ccaactcttt ttccgaaggt aactggcttc agcagagcgc 5100
   agataccaaa tactgtcctt ctagtgtagc cgtagttagg ccaccacttc aagaactctg 5160
   tagcaccgcc tacatacctc gctctgctaa tcctgttacc agtggctgct gccagtggcg 5220
   ataagtcgtg tcttaccggg ttggactcaa gacgatagtt accggataag gcgcagcggt 5280
   cgggctgaac ggggggttcg tgcacacagc ccagcttgga gcgaacgacc tacaccgaac 5340
   tgagatacct acagcgtgag ctatgagaaa gcgccacgct tcccgaaggg agaaaggcgg 5400
   acaggtatcc ggtaagcggc agggtcggaa caggagagcg cacgagggag cttccagggg 5460
   gaaacgcctg gtatctttat agtcctgtcg ggtttcgcca cctctgactt gagcgtcgat 5520
   ttttgtgatg ctcgtcaggg gggcggagcc tatggaaaaa cgccagcaac gcggcctttt 5580
   tacggttcct ggccttttgc tggccttttg ctcacatgtt ctttcctgcg ttatcccctg 5640
   attctgtgga taaccgtatt accgcctttg agtgagctga taccgctcgc cgcagccgaa 5700
   cgaccgagcg cagcgagtca gtgagcgagg aagcggaaga gcgcccaata cgcaaaccgc 5760
   ctctccccgc gcgttggccg attcattaat gcagctggca cgacaggttt cccgactgga 5820
   aagcgggcag tgagcgcaac gcaattaatg tgagttagct cactcattag gcaccccagg 5880
   ctttacactt tatgcttccg gctcgtatgt tgtgtggaat tgtgagcgga taacaatttc 5940
   acacaggaaa cagctatgac catgattacg ccaagctttg gctaacacac acgccattcc 6000
   aaccaatagt tttctcggca taaagccatg ctctgacgct taaatgcact aatgccttaa 6060
   aaaaacatta aagtctaaca cactagactt atttacttcg taattaagtc gttaaaccgt 6120
   gtgctctacg accaaaagta taaaaccttt aagaactttc ttttttcttg taaaaaaaga 6180
   aactagataa atctctcata tcttttattc aataatcgca tcagattgca gtataaattt 6240
   aacgatcact catcatgttc atatttatca gagctcgtgc tataattata ctaattttat 6300
   aaggaggaaa aaataaagag ggttataatg aacgagaaaa atataaaaca cagtcaaaac 6360
   tttattactt caaaacataa tatagataaa ataatgacaa atataagatt aaatgaacat 6420
   gataatatct ttgaaatcgg ctcaggaaaa gggcatttta cccttgaatt agtacagagg 6480
   tgtaatttcg taactgccat tgaaatagac cataaattat gcaaaactac agaaaataaa 6540
   cttgttgatc acgataattt ccaagtttta aacaaggata tattgcagtt taaatttcct 6600
   aaaaaccaat cctataaaat atttggtaat ataccttata acataagtac ggatataata 6660
   cgcaaaattg tttttgatag tatagctgat gagatttatt taatcgtgga atacgggttt 6720
   gctaaaagat tattaaatac aaaacgctca ttggcattat ttttaatggc agaagttgat 6780
   atttctatat taagtatggt tccaagagaa tattttcatc ctaaacctaa agtgaatagc 6840
   tcacttatca gattaaatag aaaaaaatca agaatatcac acaaagataa acagaagtat 6900
   aattatttcg ttatgaaatg ggttaacaaa gaatacaaga aaatatttac aaaaaatcaa 6960
   tttaacaatt ccttaaaaca tgcaggaatt gacgatttaa acaatattag ctttgaacaa 7020
   ttcttatctc ttttcaatag ctataaatta tttaataagt aagttaaggg atgcataaac 7080
   tgcatccctt aacttgtttt tcgtgtacct attttttgtg aatcgattat gtcttttgcg 7140
   cattcacttc ttttctatat aaatatgagc gaagcgaata agcgtcggaa aagcagcaaa 7200
   aagtttcctt tttgctgttg gagcatgggg gttcaggggg tgcagtatct gacgtcaatg 7260
   ccgagcgaaa gcgagccgaa gggtagcatt tacgttagat aaccccctga tatgctccga 7320
   cgctttatat agaaaagaag attcaactag gtaaaatctt aatataggtt gagatgataa 7380
   ggtttataag gaatttgttt gttctaattt ttcactcatt ttgttctaat ttcttttaac 7440
   aaatgttctt ttttttttag aacagttatg atatagttag aatagtttaa aataaggagt 7500
   gagaaaaaga tgaaagaaag atatggaaca gtctataaag gctctcagag gctcatagac 7560
   gaagaaagtg gagaagtcat agaggtagac aagttatacc gtaaacaaac gtctggtaac 7620
   ttcgtaaagg catatatagt gcaattaata agtatgttag atatgattgg cggaaaaaaa   7680
   cttaaaatcg ttaactatat cctagataat gtccacttaa gtaacaatac aatgatagct   7740
   acaacaagag aaatagcaaa agctacagga acaagtctac aaacagtaat aacaacactt   7800
   aaaatcttag aagaaggaaa tattataaaa agaaaaactg gagtattaat gttaaaccct   7860
   gaactactaa tgagaggcga cgaccaaaaa caaaaatacc tcttactcga atttgggaac   7920
   tttgagcaag aggcaaatga aatagattga cctcccaata acaccacgta gttattggga   7980
   ggtcaatcta tgaaaatgcg atta                                          8004
<210> 16
   <211> 7693
   <212> DNA
   <213> Artificial
<220>
   <223> Plasmid
<400> 16
   agcttggctg caggtcgact tcaagaagag gcacctcatc ttggaaagcc tgtacttgtt     60
   ttaagagatg ttactgaaag accagaagca gttgaggcag gaacggtaaa attagtagga    120
   actgatatta aaaagatagt agacgaagcc tataaaataa tgaaagatga agaagaatat    180
   gaaaaaatga gtaaggcaat aaatccatat ggtgatggaa aggcttcaga tagaatagtt    240
   gatgctatat tgtatcattt tggagtatta aaggatagac cagatgaatt ttcacctaaa    300
   aagtaataca tgagcattct aaaagaattt agaatgaagt ttcttatgca caagtatttt    360
   ttattacatt aatatagtta aaatataaac ttatgtattt atgctaaaac atgattttaa    420
   gggggttagc atatgcataa gtttaatttt tttgttaaaa aatattaaac tttgtgtttt    480
   ttttaacaaa atatattgat aaaaataata atagtgggta taattaagtt gttagagaaa    540
   acgtataaat tagggataaa ctatggaact tatgaaatag attgaaatgg tttatctgtt    600
   accccgtatc aaaatttagg aggttagtta gaatgaaaga agttgtaata gctagtgcag    660
   taagaacagc gattggatct tatggaaagt ctcttaagga tgtaccagca gtagatttag    720
   gagctacagc tataaaggaa gcagttaaaa aagcaggaat aaaaccagag gatgttaatg    780
   aagtcatttt aggaaatgtt cttcaagcag gtttaggaca gaatccagca agacaggcat    840
   cttttaaagc aggattacca gttgaaattc cagctatgac tattaataag gtttgtggtt    900
   caggacttag aacagttagc ttagcagcac aaattataaa agcaggagat gctgacgtaa    960
   taatagcagg tggtatggaa aatatgtcta gagctcctta cttagcgaat aacgctagat   1020
   ggggatatag aatgggaaac gctaaatttg ttgatgaaat gatcactgac ggattgtggg   1080
   atgcatttaa tgattaccac atgggaataa cagcagaaaa catagctgag agatggaaca   1140
   tttcaagaga agaacaagat gagtttgctc ttgcatcaca aaaaaaagct gaagaagcta   1200
   taaaatcagg tcaatttaaa gatgaaatag ttcctgtagt aattaaaggc agaaagggag   1260
   aaactgtagt tgatacagat gagcacccta gatttggatc aactatagaa ggacttgcaa   1320
   aattaaaacc tgccttcaaa aaagatggaa cagttacagc tggtaatgca tcaggattaa   1380
   atgactgtgc agcagtactt gtaatcatga gtgcagaaaa agctaaagag cttggagtaa   1440
   aaccacttgc taagatagtt tcttatggtt cagcaggagt tgacccagca ataatgggat   1500
   atggaccttt ctatgcaaca aaagcagcta ttgaaaaagc aggttggaca gttgatgaat   1560
   tagatttaat agaatcaaat gaagcttttg cagctcaaag tttagcagta gcaaaagatt   1620
   taaaatttga tatgaataaa gtaaatgtaa atggaggagc tattgccctt ggtcatccaa   1680
   ttggagcatc aggtgcaaga atactcgtta ctcttgtaca cgcaatgcaa aaaagagatg   1740
   caaaaaaagg cttagcaact ttatgtatag gtggcggaca aggaacagca atattgctag   1800
   aaaagtgcta gaaaggatcc tgtttaactt taagaaggag atatacatat gttggataat   1860
   ggcttttctt ttcctgttcg tgtgtattat gaagatactg atgcaggtgg cgtagtgtat 1920
   cacgctcgct atttgcattt ttttgaacga gcaagaacag aatatttgcg tacattaaat 1980
   tttacgcaac aaaccttact agaggaacaa caactcgcat ttgttgtcaa aacgctcgcc 2040
   attgattatt gcgtggcagc aaaattggat gatttactta tggtggaaac agaggtttca 2100
   gaagtaaaag gggctacaat cctttttgaa cagagactga tgcgcaacac cctgatgtta 2160
   tcaaaggcta ctgttaaggt agcctgtgtt gatctaggca agatgaaacc agtggcgttt 2220
   cccaaagaag ttaaagcggc gtttcatcac ttaaaataat ttttcggagt atgcaatgag 2280
   gtacctttta tgttaaagga tgaagtaatt aaacaaatta gcacgccatt aacttcgcct 2340
   gcatttccta gaggacccta taaatttcat aatcgtgagt attttaacat tgtatatcgt 2400
   acagatatgg atgcacttcg taaagttgtg ccagagcctt tagaaattga tgagccctta 2460
   gtcaggtttg aaattatggc aatgcatgat acgagtggac ttggttgtta tacagaaagc 2520
   ggacaggcta ttcccgtaag ctttaatgga gttaagggag attatcttca tatgatgtat 2580
   ttagataatg agcctgcaat tgcagtagga agggaattaa gtgcatatcc taaaaagctc 2640
   gggtatccaa agctttttgt ggattcagat actttagtag gaactttaga ctatggaaaa 2700
   cttagagttg cgacagctac aatggggtac aaacataaag ccttagatgc taatgaagca 2760
   aaggatcaaa tttgtcgccc taattatatg ttgaaaataa tacccaatta tgatggaagc 2820
   cctagaatat gtgagcttat aaatgcgaaa atcacagatg ttaccgtaca tgaagcttgg 2880
   acaggaccaa ctcgactgca gttatttgat cacgctatgg cgccacttaa tgatttgcca 2940
   gtaaaagaga ttgtttctag ctctcacatt cttgcagata taatattgcc tagagctgaa 3000
   gttatatatg attatcttaa gtaatagaat tcactggccg tcgttttaca acgtcgtgac 3060
   tgggaaaacc ctggcgttac ccaacttaat cgccttgcag cacatccccc tttcgccagc 3120
   tggcgtaata gcgaagaggc ccgcaccgat cgcccttccc aacagttgcg cagcctgaat 3180
   ggcgaatggc gcctgatgcg gtattttctc cttacgcatc tgtgcggtat ttcacaccgc 3240
   atatggtgca ctctcagtac aatctgctct gatgccgcat agttaagcca gccccgacac 3300
   ccgccaacac ccgctgacgc gccctgacgg gcttgtctgc tcccggcatc cgcttacaga 3360
   caagctgtga ccgtctccgg gagctgcatg tgtcagaggt tttcaccgtc atcaccgaaa 3420
   cgcgcgagac gaaagggcct cgtgatacgc ctatttttat aggttaatgt catgataata 3480
   atggtttctt agacgtcagg tggcactttt cggggaaatg tgcgcggaac ccctatttgt 3540
   ttatttttct aaatacattc aaatatgtat ccgctcatga gacaataacc ctgataaatg 3600
   cttcaataat attgaaaaag gaagagtatg agtattcaac atttccgtgt cgcccttatt 3660
   cccttttttg cggcattttg ccttcctgtt tttgctcacc cagaaacgct ggtgaaagta 3720
   aaagatgctg aagatcagtt gggtgcacga gtgggttaca tcgaactgga tctcaacagc 3780
   ggtaagatcc ttgagagttt tcgccccgaa gaacgttttc caatgatgag cacttttaaa 3840
   gttctgctat gtggcgcggt attatcccgt attgacgccg ggcaagagca actcggtcgc 3900
   cgcatacact attctcagaa tgacttggtt gagtactcac cagtcacaga aaagcatctt 3960
   acggatggca tgacagtaag agaattatgc agtgctgcca taaccatgag tgataacact 4020
   gcggccaact tacttctgac aacgatcgga ggaccgaagg agctaaccgc ttttttgcac 4080
   aacatggggg atcatgtaac tcgccttgat cgttgggaac cggagctgaa tgaagccata 4140
   ccaaacgacg agcgtgacac cacgatgcct gtagcaatgg caacaacgtt gcgcaaacta 4200
   ttaactggcg aactacttac tctagcttcc cggcaacaat taatagactg gatggaggcg 4260
   gataaagttg caggaccact tctgcgctcg gcccttccgg ctggctggtt tattgctgat 4320
   aaatctggag ccggtgagcg tgggtctcgc ggtatcattg cagcactggg gccagatggt 4380
   aagccctccc gtatcgtagt tatctacacg acggggagtc aggcaactat ggatgaacga 4440
   aatagacaga tcgctgagat aggtgcctca ctgattaagc attggtaact gtcagaccaa 4500
   gtttactcat atatacttta gattgattta aaacttcatt tttaatttaa aaggatctag 4560
   gtgaagatcc tttttgataa tctcatgacc aaaatccctt aacgtgagtt ttcgttccac 4620
   tgagcgtcag accccgtaga aaagatcaaa ggatcttctt gagatccttt ttttctgcgc 4680
   gtaatctgct gcttgcaaac aaaaaaacca ccgctaccag cggtggtttg tttgccggat 4740
   caagagctac caactctttt tccgaaggta actggcttca gcagagcgca gataccaaat 4800
   actgtccttc tagtgtagcc gtagttaggc caccacttca agaactctgt agcaccgcct 4860
   acatacctcg ctctgctaat cctgttacca gtggctgctg ccagtggcga taagtcgtgt 4920
   cttaccgggt tggactcaag acgatagtta ccggataagg cgcagcggtc gggctgaacg 4980
   gggggttcgt gcacacagcc cagcttggag cgaacgacct acaccgaact gagataccta 5040
   cagcgtgagc tatgagaaag cgccacgctt cccgaaggga gaaaggcgga caggtatccg 5100
   gtaagcggca gggtcggaac aggagagcgc acgagggagc ttccaggggg aaacgcctgg 5160
   tatctttata gtcctgtcgg gtttcgccac ctctgacttg agcgtcgatt tttgtgatgc 5220
   tcgtcagggg ggcggagcct atggaaaaac gccagcaacg cggccttttt acggttcctg 5280
   gccttttgct ggccttttgc tcacatgttc tttcctgcgt tatcccctga ttctgtggat 5340
   aaccgtatta ccgcctttga gtgagctgat accgctcgcc gcagccgaac gaccgagcgc 5400
   agcgagtcag tgagcgagga agcggaagag cgcccaatac gcaaaccgcc tctccccgcg 5460
   cgttggccga ttcattaatg cagctggcac gacaggtttc ccgactggaa agcgggcagt 5520
   gagcgcaacg caattaatgt gagttagctc actcattagg caccccaggc tttacacttt 5580
   atgcttccgg ctcgtatgtt gtgtggaatt gtgagcggat aacaatttca cacaggaaac 5640
   agctatgacc atgattacgc caagctttgg ctaacacaca cgccattcca accaatagtt 5700
   ttctcggcat aaagccatgc tctgacgctt aaatgcacta atgccttaaa aaaacattaa 5760
   agtctaacac actagactta tttacttcgt aattaagtcg ttaaaccgtg tgctctacga 5820
   ccaaaagtat aaaaccttta agaactttct tttttcttgt aaaaaaagaa actagataaa 5880
   tctctcatat cttttattca ataatcgcat cagattgcag tataaattta acgatcactc 5940
   atcatgttca tatttatcag agctcgtgct ataattatac taattttata aggaggaaaa 6000
   aataaagagg gttataatga acgagaaaaa tataaaacac agtcaaaact ttattacttc 6060
   aaaacataat atagataaaa taatgacaaa tataagatta aatgaacatg ataatatctt 6120
   tgaaatcggc tcaggaaaag ggcattttac ccttgaatta gtacagaggt gtaatttcgt 6180
   aactgccatt gaaatagacc ataaattatg caaaactaca gaaaataaac ttgttgatca 6240
   cgataatttc caagttttaa acaaggatat attgcagttt aaatttccta aaaaccaatc 6300
   ctataaaata tttggtaata taccttataa cataagtacg gatataatac gcaaaattgt 6360
   ttttgatagt atagctgatg agatttattt aatcgtggaa tacgggtttg ctaaaagatt 6420
   attaaataca aaacgctcat tggcattatt tttaatggca gaagttgata tttctatatt 6480
   aagtatggtt ccaagagaat attttcatcc taaacctaaa gtgaatagct cacttatcag 6540
   attaaataga aaaaaatcaa gaatatcaca caaagataaa cagaagtata attatttcgt 6600
   tatgaaatgg gttaacaaag aatacaagaa aatatttaca aaaaatcaat ttaacaattc 6660
   cttaaaacat gcaggaattg acgatttaaa caatattagc tttgaacaat tcttatctct 6720
   tttcaatagc tataaattat ttaataagta agttaaggga tgcataaact gcatccctta 6780
   acttgttttt cgtgtaccta ttttttgtga atcgattatg tcttttgcgc attcacttct 6840
   tttctatata aatatgagcg aagcgaataa gcgtcggaaa agcagcaaaa agtttccttt 6900
   ttgctgttgg agcatggggg ttcagggggt gcagtatctg acgtcaatgc cgagcgaaag 6960
   cgagccgaag ggtagcattt acgttagata accccctgat atgctccgac gctttatata 7020
   gaaaagaaga ttcaactagg taaaatctta atataggttg agatgataag gtttataagg 7080
   aatttgtttg ttctaatttt tcactcattt tgttctaatt tcttttaaca aatgttcttt 7140
   tttttttaga acagttatga tatagttaga atagtttaaa ataaggagtg agaaaaagat 7200
   gaaagaaaga tatggaacag tctataaagg ctctcagagg ctcatagacg aagaaagtgg 7260
   agaagtcata gaggtagaca agttataccg taaacaaacg tctggtaact tcgtaaaggc 7320
   atatatagtg caattaataa gtatgttaga tatgattggc ggaaaaaaac ttaaaatcgt 7380
   taactatatc ctagataatg tccacttaag taacaataca atgatagcta caacaagaga   7440
   aatagcaaaa gctacaggaa caagtctaca aacagtaata acaacactta aaatcttaga   7500
   agaaggaaat attataaaaa gaaaaactgg agtattaatg ttaaaccctg aactactaat   7560
   gagaggcgac gaccaaaaac aaaaatacct cttactcgaa tttgggaact ttgagcaaga   7620
   ggcaaatgaa atagattgac ctcccaataa caccacgtag ttattgggag gtcaatctat   7680
   gaaaatgcga tta                                                     7693

### SEQUENCE LISTING

<110> Evonik Degussa
<120> Zellen und Verfahren zur Herstellung von Aceton
<130> 200900033
<160> 16
<170> PatentIn version 3.4
<210> 1
   <211> 17
   <212> DNA
   <213> Artificial
<220>
   <223> Primer
<400> 1
   ggaaggtacc ttttatg 17
<210> 2
   <211> 25
   <212> DNA
   <213> Artificial
<220>
   <223> Primer
<400> 2
   gtaactctga attctattac ttaag 25
<210> 3
   <211> 20
   <212> DNA
   <213> Artificial
<220>
   <223> Primer
<400> 3
   cacaacggtg gatccaagag 20
<210> 4
   <211> 20
   <212> DNA
   <213> Artificial
<220>
   <223> Primer
<400> 4
   cgcgatatgg taccaatcat 20
<210> 5
   <211> 28
   <212> DNA
   <213> Artificial
<220>
   <223> Primer
<400> 5
   gaatttaaaa ggagggatcc aaatgaac 28
<210> 6
   <211> 27
   <212> DNA
   <213> Artificial
<220>
   <223> Primer
<400> 6
   gtttcatagt attggtacct aaacagc 27
<210> 7
   <211> 20
   <212> DNA
   <213> Artificial
<220>
   <223> Primer
<400> 7
   ctcaggtcga cttcaagaag 20
<210> 8
   <211> 29
   <212> DNA
   <213> Artificial
<220>
   <223> Primer
<400> 8
   cagagttatt tttaaggatc ctttctagc 29
<210> 9
   <211> 30
   <212> DNA
   <213> Artificial
<220>
   <223> Primer
<400> 9
   caattgggat ccgataacaa tttcacacag 30
<210> 10
   <211> 29
   <212> DNA
   <213> Artificial
<220>
   <223> Primer
<400> 10
   gagatctggt acccggttaa atgatcgga 29
<210> 11
   <211> 28
   <212> DNA
   <213> Artificial
<220>
   <223> Primer
<400> 11
   ctctagaagg atcctgttta actttaag 28
<210> 12
   <211> 25
   <212> DNA
   <213> Artificial
<220>
   <223> Primer
<400> 12
   attgggtacc tcattgcata ctccg 25
<210> 13
   <211> 8559
   <212> DNA
   <213> Artificial
<220>
   <223> Plasmid
<400> 13
<210> 14
   <211> 8563
   <212> DNA
   <213> Artificial
<220>
   <223> Plasmid
<400> 14
   agcttggctg caggtcgact tcaagaagag gcacctcatc ttggaaagcc tgtacttgtt      60
   ttaagagatg ttactgaaag accagaagca gttgaggcag gaacggtaaa attagtagga     120
   actgatatta aaaagatagt agacgaagcc tataaaataa tgaaagatga agaagaatat     180
   gaaaaaatga gtaaggcaat aaatccatat ggtgatggaa aggcttcaga tagaatagtt     240
   gatgctatat tgtatcattt tggagtatta aaggatagac cagatgaatt ttcacctaaa     300
   aagtaataca tgagcattct aaaagaattt agaatgaagt ttcttatgca caagtatttt     360
   ttattacatt aatatagtta aaatataaac ttatgtattt atgctaaaac atgattttaa     420
   gggggttagc atatgcataa gtttaatttt tttgttaaaa aatattaaac tttgtgtttt     480
   ttttaacaaa atatattgat aaaaataata atagtgggta taattaagtt gttagagaaa     540
   acgtataaat tagggataaa ctatggaact tatgaaatag attgaaatgg tttatctgtt     600
   accccgtatc aaaatttagg aggttagtta gaatgaaaga agttgtaata gctagtgcag     660
   taagaacagc gattggatct tatggaaagt ctcttaagga tgtaccagca gtagatttag     720
   gagctacagc tataaaggaa gcagttaaaa aagcaggaat aaaaccagag gatgttaatg     780
   aagtcatttt aggaaatgtt cttcaagcag gtttaggaca gaatccagca agacaggcat     840
   cttttaaagc aggattacca gttgaaattc cagctatgac tattaataag gtttgtggtt     900
   caggacttag aacagttagc ttagcagcac aaattataaa agcaggagat gctgacgtaa     960
   taatagcagg tggtatggaa aatatgtcta gagctcctta cttagcgaat aacgctagat    1020
   ggggatatag aatgggaaac gctaaatttg ttgatgaaat gatcactgac ggattgtggg    1080
   atgcatttaa tgattaccac atgggaataa cagcagaaaa catagctgag agatggaaca    1140
   tttcaagaga agaacaagat gagtttgctc ttgcatcaca aaaaaaagct gaagaagcta    1200
   taaaatcagg tcaatttaaa gatgaaatag ttcctgtagt aattaaaggc agaaagggag    1260
   aaactgtagt tgatacagat gagcacccta gatttggatc aactatagaa ggacttgcaa    1320
   aattaaaacc tgccttcaaa aaagatggaa cagttacagc tggtaatgca tcaggattaa 1380
   atgactgtgc agcagtactt gtaatcatga gtgcagaaaa agctaaagag cttggagtaa 1440
   aaccacttgc taagatagtt tcttatggtt cagcaggagt tgacccagca ataatgggat 1500
   atggaccttt ctatgcaaca aaagcagcta ttgaaaaagc aggttggaca gttgatgaat 1560
   tagatttaat agaatcaaat gaagcttttg cagctcaaag tttagcagta gcaaaagatt 1620
   taaaatttga tatgaataaa gtaaatgtaa atggaggagc tattgccctt ggtcatccaa 1680
   ttggagcatc aggtgcaaga atactcgtta ctcttgtaca cgcaatgcaa aaaagagatg 1740
   caaaaaaagg cttagcaact ttatgtatag gtggcggaca aggaacagca atattgctag 1800
   aaaagtgcta gaaaggatcc aagagggata aaaaatgaaa acaaaattga tgacattaca 1860
   agacgccacc ggcttctttc gtgacggcat gaccatcatg gtgggcggat ttatggggat 1920
   tggcactcca tcccgcctgg ttgaagcatt actggaatct ggtgttcgcg acctgacatt 1980
   gatagccaat gataccgcgt ttgttgatac cggcatcggt ccgctcatcg tcaatggtcg 2040
   agtccgcaaa gtgattgctt cacatatcgg caccaacccg gaaacaggtc ggcgcatgat 2100
   atctggtgag atggacgtcg ttctggtgcc gcaaggtacg ctaatcgagc aaattcgctg 2160
   tggtggagct ggacttggtg gttttctcac cccaacgggt gtcggcaccg tcgtagagga 2220
   aggcaaacag acactgacac tcgacggtaa aacctggctg ctcgaacgcc cactgcgcgc 2280
   cgacctggcg ctaattcgcg ctcatcgttg cgacacactt ggcaacctga cctatcaact 2340
   tagcgcccgc aactttaacc ccctgatagc ccttgcggct gatatcacgc tggtagagcc 2400
   agatgaactg gtcgaaaccg gcgagctgca acctgaccat attgtcaccc ctggtgccgt 2460
   tatcgaccac atcatcgttt cacaggagag caaataatgg atgcgaaaca acgtattgcg 2520
   cgccgtgtgg cgcaagagct tcgtgatggt gacatcgtta acttagggat cggtttaccc 2580
   acaatggtcg ccaattattt accggagggt attcatatca ctctgcaatc ggaaaacggc 2640
   ttcctcggtt taggcccggt cacgacagcg catccagatc tggtgaacgc tggcgggcaa 2700
   ccgtgcggtg ttttacccgg tgcagccatg tttgatagcg ccatgtcatt tgcgctaatc 2760
   cgtggcggtc atattgatgc ctgcgtgctc ggcggtttgc aagtagacga agaagcaaac 2820
   ctcgcgaact gggtagtgcc tgggaaaatg gtgcccggta tgggtggcgc gatggatctg 2880
   gtgaccgggt cgcgcaaagt gatcatcgcc atggaacatt gcgccaaaga tggttcagca 2940
   aaaattttgc gccgctgcac catgccactc actgcgcaac atgcggtgca tatgctggtt 3000
   actgaactgg ctgtctttcg ttttattgac ggcaaaatgt ggctcaccga aattgccgac 3060
   gggtgtgatt tagccaccgt gcgtgccaaa acagaagctc ggtttgaagt cgccgccgat 3120
   ctgaatacgc aacggggtga tttatgattg gtacctttta tgttaaagga tgaagtaatt 3180
   aaacaaatta gcacgccatt aacttcgcct gcatttccta gaggacccta taaatttcat 3240
   aatcgtgagt attttaacat tgtatatcgt acagatatgg atgcacttcg taaagttgtg 3300
   ccagagcctt tagaaattga tgagccctta gtcaggtttg aaattatggc aatgcatgat 3360
   acgagtggac ttggttgtta tacagaaagc ggacaggcta ttcccgtaag ctttaatgga 3420
   gttaagggag attatcttca tatgatgtat ttagataatg agcctgcaat tgcagtagga 3480
   agggaattaa gtgcatatcc taaaaagctc gggtatccaa agctttttgt ggattcagat 3540
   actttagtag gaactttaga ctatggaaaa cttagagttg cgacagctac aatggggtac 3600
   aaacataaag ccttagatgc taatgaagca aaggatcaaa tttgtcgccc taattatatg 3660
   ttgaaaataa tacccaatta tgatggaagc cctagaatat gtgagcttat aaatgcgaaa 3720
   atcacagatg ttaccgtaca tgaagcttgg acaggaccaa ctcgactgca gttatttgat 3780
   cacgctatgg cgccacttaa tgatttgcca gtaaaagaga ttgtttctag ctctcacatt 3840
   cttgcagata taatattgcc tagagctgaa gttatatatg attatcttaa gtaatagaat 3900
   tcactggccg tcgttttaca acgtcgtgac tgggaaaacc ctggcgttac ccaacttaat 3960
   cgccttgcag cacatccccc tttcgccagc tggcgtaata gcgaagaggc ccgcaccgat 4020
   cgcccttccc aacagttgcg cagcctgaat ggcgaatggc gcctgatgcg gtattttctc 4080
   cttacgcatc tgtgcggtat ttcacaccgc atatggtgca ctctcagtac aatctgctct 4140
   gatgccgcat agttaagcca gccccgacac ccgccaacac ccgctgacgc gccctgacgg 4200
   gcttgtctgc tcccggcatc cgcttacaga caagctgtga ccgtctccgg gagctgcatg 4260
   tgtcagaggt tttcaccgtc atcaccgaaa cgcgcgagac gaaagggcct cgtgatacgc 4320
   ctatttttat aggttaatgt catgataata atggtttctt agacgtcagg tggcactttt 4380
   cggggaaatg tgcgcggaac ccctatttgt ttatttttct aaatacattc aaatatgtat 4440
   ccgctcatga gacaataacc ctgataaatg cttcaataat attgaaaaag gaagagtatg 4500
   agtattcaac atttccgtgt cgcccttatt cccttttttg cggcattttg ccttcctgtt 4560
   tttgctcacc cagaaacgct ggtgaaagta aaagatgctg aagatcagtt gggtgcacga 4620
   gtgggttaca tcgaactgga tctcaacagc ggtaagatcc ttgagagttt tcgccccgaa 4680
   gaacgttttc caatgatgag cacttttaaa gttctgctat gtggcgcggt attatcccgt 4740
   attgacgccg ggcaagagca actcggtcgc cgcatacact attctcagaa tgacttggtt 4800
   gagtactcac cagtcacaga aaagcatctt acggatggca tgacagtaag agaattatgc 4860
   agtgctgcca taaccatgag tgataacact gcggccaact tacttctgac aacgatcgga 4920
   ggaccgaagg agctaaccgc ttttttgcac aacatggggg atcatgtaac tcgccttgat 4980
   cgttgggaac cggagctgaa tgaagccata ccaaacgacg agcgtgacac cacgatgcct 5040
   gtagcaatgg caacaacgtt gcgcaaacta ttaactggcg aactacttac tctagcttcc 5100
   cggcaacaat taatagactg gatggaggcg gataaagttg caggaccact tctgcgctcg 5160
   gcccttccgg ctggctggtt tattgctgat aaatctggag ccggtgagcg tgggtctcgc 5220
   ggtatcattg cagcactggg gccagatggt aagccctccc gtatcgtagt tatctacacg 5280
   acggggagtc aggcaactat ggatgaacga aatagacaga tcgctgagat aggtgcctca 5340
   ctgattaagc attggtaact gtcagaccaa gtttactcat atatacttta gattgattta 5400
   aaacttcatt tttaatttaa aaggatctag gtgaagatcc tttttgataa tctcatgacc 5460
   aaaatccctt aacgtgagtt ttcgttccac tgagcgtcag accccgtaga aaagatcaaa 5520
   ggatcttctt gagatccttt ttttctgcgc gtaatctgct gcttgcaaac aaaaaaacca 5580
   ccgctaccag cggtggtttg tttgccggat caagagctac caactctttt tccgaaggta 5640
   actggcttca gcagagcgca gataccaaat actgtccttc tagtgtagcc gtagttaggc 5700
   caccacttca agaactctgt agcaccgcct acatacctcg ctctgctaat cctgttacca 5760
   gtggctgctg ccagtggcga taagtcgtgt cttaccgggt tggactcaag acgatagtta 5820
   ccggataagg cgcagcggtc gggctgaacg gggggttcgt gcacacagcc cagcttggag 5880
   cgaacgacct acaccgaact gagataccta cagcgtgagc tatgagaaag cgccacgctt 5940
   cccgaaggga gaaaggcgga caggtatccg gtaagcggca gggtcggaac aggagagcgc 6000
   acgagggagc ttccaggggg aaacgcctgg tatctttata gtcctgtcgg gtttcgccac 6060
   ctctgacttg agcgtcgatt tttgtgatgc tcgtcagggg ggcggagcct atggaaaaac 6120
   gccagcaacg cggccttttt acggttcctg gccttttgct ggccttttgc tcacatgttc 6180
   tttcctgcgt tatcccctga ttctgtggat aaccgtatta ccgcctttga gtgagctgat 6240
   accgctcgcc gcagccgaac gaccgagcgc agcgagtcag tgagcgagga agcggaagag 6300
   cgcccaatac gcaaaccgcc tctccccgcg cgttggccga ttcattaatg cagctggcac 6360
   gacaggtttc ccgactggaa agcgggcagt gagcgcaacg caattaatgt gagttagctc 6420
   actcattagg caccccaggc tttacacttt atgcttccgg ctcgtatgtt gtgtggaatt 6480
   gtgagcggat aacaatttca cacaggaaac agctatgacc atgattacgc caagctttgg 6540
   ctaacacaca cgccattcca accaatagtt ttctcggcat aaagccatgc tctgacgctt 6600
   aaatgcacta atgccttaaa aaaacattaa agtctaacac actagactta tttacttcgt 6660
   aattaagtcg ttaaaccgtg tgctctacga ccaaaagtat aaaaccttta agaactttct 6720
   tttttcttgt aaaaaaagaa actagataaa tctctcatat cttttattca ataatcgcat 6780
   cagattgcag tataaattta acgatcactc atcatgttca tatttatcag agctcgtgct 6840
   ataattatac taattttata aggaggaaaa aataaagagg gttataatga acgagaaaaa 6900
   tataaaacac agtcaaaact ttattacttc aaaacataat atagataaaa taatgacaaa 6960
   tataagatta aatgaacatg ataatatctt tgaaatcggc tcaggaaaag ggcattttac 7020
   ccttgaatta gtacagaggt gtaatttcgt aactgccatt gaaatagacc ataaattatg 7080
   caaaactaca gaaaataaac ttgttgatca cgataatttc caagttttaa acaaggatat 7140
   attgcagttt aaatttccta aaaaccaatc ctataaaata tttggtaata taccttataa 7200
   cataagtacg gatataatac gcaaaattgt ttttgatagt atagctgatg agatttattt 7260
   aatcgtggaa tacgggtttg ctaaaagatt attaaataca aaacgctcat tggcattatt 7320
   tttaatggca gaagttgata tttctatatt aagtatggtt ccaagagaat attttcatcc 7380
   taaacctaaa gtgaatagct cacttatcag attaaataga aaaaaatcaa gaatatcaca 7440
   caaagataaa cagaagtata attatttcgt tatgaaatgg gttaacaaag aatacaagaa 7500
   aatatttaca aaaaatcaat ttaacaattc cttaaaacat gcaggaattg acgatttaaa 7560
   caatattagc tttgaacaat tcttatctct tttcaatagc tataaattat ttaataagta 7620
   agttaaggga tgcataaact gcatccctta acttgttttt cgtgtaccta ttttttgtga 7680
   atcgattatg tcttttgcgc attcacttct tttctatata aatatgagcg aagcgaataa 7740
   gcgtcggaaa agcagcaaaa agtttccttt ttgctgttgg agcatggggg ttcagggggt 7800
   gcagtatctg acgtcaatgc cgagcgaaag cgagccgaag ggtagcattt acgttagata 7860
   accccctgat atgctccgac gctttatata gaaaagaaga ttcaactagg taaaatctta 7920
   atataggttg agatgataag gtttataagg aatttgtttg ttctaatttt tcactcattt 7980
   tgttctaatt tcttttaaca aatgttcttt tttttttaga acagttatga tatagttaga 8040
   atagtttaaa ataaggagtg agaaaaagat gaaagaaaga tatggaacag tctataaagg 8100
   ctctcagagg ctcatagacg aagaaagtgg agaagtcata gaggtagaca agttataccg 8160
   taaacaaacg tctggtaact tcgtaaaggc atatatagtg caattaataa gtatgttaga 8220
   tatgattggc ggaaaaaaac ttaaaatcgt taactatatc ctagataatg tccacttaag 8280
   taacaataca atgatagcta caacaagaga aatagcaaaa gctacaggaa caagtctaca 8340
   aacagtaata acaacactta aaatcttaga agaaggaaat attataaaaa gaaaaactgg 8400
   agtattaatg ttaaaccctg aactactaat gagaggcgac gaccaaaaac aaaaatacct 8460
   cttactcgaa tttgggaact ttgagcaaga ggcaaatgaa atagattgac ctcccaataa 8520
   caccacgtag ttattgggag gtcaatctat gaaaatgcga tta 8563
<210> 15
   <211> 8004
   <212> DNA
   <213> Artificial
<220>
   <223> Plasmid
<400> 15
   agcttggctg caggtcgact tcaagaagag gcacctcatc ttggaaagcc tgtacttgtt 60
   ttaagagatg ttactgaaag accagaagca gttgaggcag gaacggtaaa attagtagga 120
   actgatatta aaaagatagt agacgaagcc tataaaataa tgaaagatga agaagaatat 180
   gaaaaaatga gtaaggcaat aaatccatat ggtgatggaa aggcttcaga tagaatagtt 240
   gatgctatat tgtatcattt tggagtatta aaggatagac cagatgaatt ttcacctaaa 300
   aagtaataca tgagcattct aaaagaattt agaatgaagt ttcttatgca caagtatttt 360
   ttattacatt aatatagtta aaatataaac ttatgtattt atgctaaaac atgattttaa 420
   gggggttagc atatgcataa gtttaatttt tttgttaaaa aatattaaac tttgtgtttt 480
   ttttaacaaa atatattgat aaaaataata atagtgggta taattaagtt gttagagaaa 540
   acgtataaat tagggataaa ctatggaact tatgaaatag attgaaatgg tttatctgtt 600
   accccgtatc aaaatttagg aggttagtta gaatgaaaga agttgtaata gctagtgcag 660
   taagaacagc gattggatct tatggaaagt ctcttaagga tgtaccagca gtagatttag 720
   gagctacagc tataaaggaa gcagttaaaa aagcaggaat aaaaccagag gatgttaatg 780
   aagtcatttt aggaaatgtt cttcaagcag gtttaggaca gaatccagca agacaggcat 840
   cttttaaagc aggattacca gttgaaattc cagctatgac tattaataag gtttgtggtt 900
   caggacttag aacagttagc ttagcagcac aaattataaa agcaggagat gctgacgtaa 960
   taatagcagg tggtatggaa aatatgtcta gagctcctta cttagcgaat aacgctagat 1020
   ggggatatag aatgggaaac gctaaatttg ttgatgaaat gatcactgac ggattgtggg 1080
   atgcatttaa tgattaccac atgggaataa cagcagaaaa catagctgag agatggaaca 1140
   tttcaagaga agaacaagat gagtttgctc ttgcatcaca aaaaaaagct gaagaagcta 1200
   taaaatcagg tcaatttaaa gatgaaatag ttcctgtagt aattaaaggc agaaagggag 1260
   aaactgtagt tgatacagat gagcacccta gatttggatc aactatagaa ggacttgcaa 1320
   aattaaaacc tgccttcaaa aaagatggaa cagttacagc tggtaatgca tcaggattaa 1380
   atgactgtgc agcagtactt gtaatcatga gtgcagaaaa agctaaagag cttggagtaa 1440
   aaccacttgc taagatagtt tcttatggtt cagcaggagt tgacccagca ataatgggat 1500
   atggaccttt ctatgcaaca aaagcagcta ttgaaaaagc aggttggaca gttgatgaat 1560
   tagatttaat agaatcaaat gaagcttttg cagctcaaag tttagcagta gcaaaagatt 1620
   taaaatttga tatgaataaa gtaaatgtaa atggaggagc tattgccctt ggtcatccaa 1680
   ttggagcatc aggtgcaaga atactcgtta ctcttgtaca cgcaatgcaa aaaagagatg 1740
   caaaaaaagg cttagcaact ttatgtatag gtggcggaca aggaacagca atattgctag 1800
   aaaagtgcta gaaaggatcc gataacaatt tcacacagaa ttcattaaag aggagaaatt 1860
   aaccatgggc caactcttca aatcatttga tgcgtcggaa aaaacaccgc tcatctgttt 1920
   tccgtttgcc ggcggctatt cggcgtcgtt tcgccctctc catgcttttt tgcaggggga 1980
   gtgcgagatg ctcgctgccg agccgccggg acacggcacg aatcaaacgt cagccattga 2040
   ggatctcgaa gagctgacgg atttgtacaa gcaagaactg aaccttcgcc ctgatcggcc 2100
   gtttgtgctg ttcggacaca gtatgggcgg aatgatcacc ttcaggctgg cgcaaaagct 2160
   tgagcgtgaa ggcatctttc cgcaggcggt tatcatttct gcaatccagc cgcctcatat 2220
   tcagcggagg aaagtgtccc acctgcctga tgatcagttt ctcgatcata ttatccaatt 2280
   aggcggaatg cccgcagagc ttgttgaaaa taaggaggtc atgtcctttt tcctgccttc 2340
   tttccgatca gattaccggg ctcttgaaca atttgagctt tacgatctgg cccagatcca 2400
   gtcgcctgtt catgtcttta acgggcttga tgataaaaaa tgcatacgag atgcggaagg 2460
   gtggaagaag tgggcaaaag acatcacatt ccatcaattt gacggcgggc acatgttcct 2520
   gctgtcacaa acggaagaag tcgcagaacg gatttttgcg atcttgaatc agcatccgat 2580
   catttaaccg ggtacctttt atgttaaagg atgaagtaat taaacaaatt agcacgccat 2640
   taacttcgcc tgcatttcct agaggaccct ataaatttca taatcgtgag tattttaaca 2700
   ttgtatatcg tacagatatg gatgcacttc gtaaagttgt gccagagcct ttagaaattg 2760
   atgagccctt agtcaggttt gaaattatgg caatgcatga tacgagtgga cttggttgtt 2820
   atacagaaag cggacaggct attcccgtaa gctttaatgg agttaaggga gattatcttc 2880
   atatgatgta tttagataat gagcctgcaa ttgcagtagg aagggaatta agtgcatatc 2940
   ctaaaaagct cgggtatcca aagctttttg tggattcaga tactttagta ggaactttag 3000
   actatggaaa acttagagtt gcgacagcta caatggggta caaacataaa gccttagatg 3060
   ctaatgaagc aaaggatcaa atttgtcgcc ctaattatat gttgaaaata atacccaatt 3120
   atgatggaag ccctagaata tgtgagctta taaatgcgaa aatcacagat gttaccgtac 3180
   atgaagcttg gacaggacca actcgactgc agttatttga tcacgctatg gcgccactta 3240
   atgatttgcc agtaaaagag attgtttcta gctctcacat tcttgcagat ataatattgc 3300
   ctagagctga agttatatat gattatctta agtaatagaa ttcactggcc gtcgttttac 3360
   aacgtcgtga ctgggaaaac cctggcgtta cccaacttaa tcgccttgca gcacatcccc 3420
   ctttcgccag ctggcgtaat agcgaagagg cccgcaccga tcgcccttcc caacagttgc 3480
   gcagcctgaa tggcgaatgg cgcctgatgc ggtattttct ccttacgcat ctgtgcggta 3540
   tttcacaccg catatggtgc actctcagta caatctgctc tgatgccgca tagttaagcc 3600
   agccccgaca cccgccaaca cccgctgacg cgccctgacg ggcttgtctg ctcccggcat 3660
   ccgcttacag acaagctgtg accgtctccg ggagctgcat gtgtcagagg ttttcaccgt 3720
   catcaccgaa acgcgcgaga cgaaagggcc tcgtgatacg cctattttta taggttaatg 3780
   tcatgataat aatggtttct tagacgtcag gtggcacttt tcggggaaat gtgcgcggaa 3840
   cccctatttg tttatttttc taaatacatt caaatatgta tccgctcatg agacaataac 3900
   cctgataaat gcttcaataa tattgaaaaa ggaagagtat gagtattcaa catttccgtg 3960
   tcgcccttat tccctttttt gcggcatttt gccttcctgt ttttgctcac ccagaaacgc 4020
   tggtgaaagt aaaagatgct gaagatcagt tgggtgcacg agtgggttac atcgaactgg 4080
   atctcaacag cggtaagatc cttgagagtt ttcgccccga agaacgtttt ccaatgatga 4140
   gcacttttaa agttctgcta tgtggcgcgg tattatcccg tattgacgcc gggcaagagc 4200
   aactcggtcg ccgcatacac tattctcaga atgacttggt tgagtactca ccagtcacag 4260
   aaaagcatct tacggatggc atgacagtaa gagaattatg cagtgctgcc ataaccatga 4320
   gtgataacac tgcggccaac ttacttctga caacgatcgg aggaccgaag gagctaaccg 4380
   cttttttgca caacatgggg gatcatgtaa ctcgccttga tcgttgggaa ccggagctga 4440
   atgaagccat accaaacgac gagcgtgaca ccacgatgcc tgtagcaatg gcaacaacgt 4500
   tgcgcaaact attaactggc gaactactta ctctagcttc ccggcaacaa ttaatagact 4560
   ggatggaggc ggataaagtt gcaggaccac ttctgcgctc ggcccttccg gctggctggt 4620
   ttattgctga taaatctgga gccggtgagc gtgggtctcg cggtatcatt gcagcactgg 4680
   ggccagatgg taagccctcc cgtatcgtag ttatctacac gacggggagt caggcaacta 4740
   tggatgaacg aaatagacag atcgctgaga taggtgcctc actgattaag cattggtaac 4800
   tgtcagacca agtttactca tatatacttt agattgattt aaaacttcat ttttaattta 4860
   aaaggatcta ggtgaagatc ctttttgata atctcatgac caaaatccct taacgtgagt 4920
   tttcgttcca ctgagcgtca gaccccgtag aaaagatcaa aggatcttct tgagatcctt 4980
   tttttctgcg cgtaatctgc tgcttgcaaa caaaaaaacc accgctacca gcggtggttt 5040
   gtttgccgga tcaagagcta ccaactcttt ttccgaaggt aactggcttc agcagagcgc 5100
   agataccaaa tactgtcctt ctagtgtagc cgtagttagg ccaccacttc aagaactctg 5160
   tagcaccgcc tacatacctc gctctgctaa tcctgttacc agtggctgct gccagtggcg 5220
   ataagtcgtg tcttaccggg ttggactcaa gacgatagtt accggataag gcgcagcggt 5280
   cgggctgaac ggggggttcg tgcacacagc ccagcttgga gcgaacgacc tacaccgaac 5340
   tgagatacct acagcgtgag ctatgagaaa gcgccacgct tcccgaaggg agaaaggcgg 5400
   acaggtatcc ggtaagcggc agggtcggaa caggagagcg cacgagggag cttccagggg 5460
   gaaacgcctg gtatctttat agtcctgtcg ggtttcgcca cctctgactt gagcgtcgat 5520
   ttttgtgatg ctcgtcaggg gggcggagcc tatggaaaaa cgccagcaac gcggcctttt 5580
   tacggttcct ggccttttgc tggccttttg ctcacatgtt ctttcctgcg ttatcccctg 5640
   attctgtgga taaccgtatt accgcctttg agtgagctga taccgctcgc cgcagccgaa 5700
   cgaccgagcg cagcgagtca gtgagcgagg aagcggaaga gcgcccaata cgcaaaccgc 5760
   ctctccccgc gcgttggccg attcattaat gcagctggca cgacaggttt cccgactgga 5820
   aagcgggcag tgagcgcaac gcaattaatg tgagttagct cactcattag gcaccccagg 5880
   ctttacactt tatgcttccg gctcgtatgt tgtgtggaat tgtgagcgga taacaatttc 5940
   acacaggaaa cagctatgac catgattacg ccaagctttg gctaacacac acgccattcc 6000
   aaccaatagt tttctcggca taaagccatg ctctgacgct taaatgcact aatgccttaa 6060
   aaaaacatta aagtctaaca cactagactt atttacttcg taattaagtc gttaaaccgt 6120
   gtgctctacg accaaaagta taaaaccttt aagaactttc ttttttcttg taaaaaaaga 6180
   aactagataa atctctcata tcttttattc aataatcgca tcagattgca gtataaattt 6240
   aacgatcact catcatgttc atatttatca gagctcgtgc tataattata ctaattttat 6300
   aaggaggaaa aaataaagag ggttataatg aacgagaaaa atataaaaca cagtcaaaac 6360
   tttattactt caaaacataa tatagataaa ataatgacaa atataagatt aaatgaacat 6420
   gataatatct ttgaaatcgg ctcaggaaaa gggcatttta cccttgaatt agtacagagg 6480
   tgtaatttcg taactgccat tgaaatagac cataaattat gcaaaactac agaaaataaa 6540
   cttgttgatc acgataattt ccaagtttta aacaaggata tattgcagtt taaatttcct 6600
   aaaaaccaat cctataaaat atttggtaat ataccttata acataagtac ggatataata 6660
   cgcaaaattg tttttgatag tatagctgat gagatttatt taatcgtgga atacgggttt 6720
   gctaaaagat tattaaatac aaaacgctca ttggcattat ttttaatggc agaagttgat 6780
   atttctatat taagtatggt tccaagagaa tattttcatc ctaaacctaa agtgaatagc 6840
   tcacttatca gattaaatag aaaaaaatca agaatatcac acaaagataa acagaagtat 6900
   aattatttcg ttatgaaatg ggttaacaaa gaatacaaga aaatatttac aaaaaatcaa 6960
   tttaacaatt ccttaaaaca tgcaggaatt gacgatttaa acaatattag ctttgaacaa 7020
   ttcttatctc ttttcaatag ctataaatta tttaataagt aagttaaggg atgcataaac 7080
   tgcatccctt aacttgtttt tcgtgtacct attttttgtg aatcgattat gtcttttgcg 7140
   cattcacttc ttttctatat aaatatgagc gaagcgaata agcgtcggaa aagcagcaaa 7200
   aagtttcctt tttgctgttg gagcatgggg gttcaggggg tgcagtatct gacgtcaatg 7260
   ccgagcgaaa gcgagccgaa gggtagcatt tacgttagat aaccccctga tatgctccga 7320
   cgctttatat agaaaagaag attcaactag gtaaaatctt aatataggtt gagatgataa 7380
   ggtttataag gaatttgttt gttctaattt ttcactcatt ttgttctaat ttcttttaac 7440
   aaatgttctt ttttttttag aacagttatg atatagttag aatagtttaa aataaggagt 7500
   gagaaaaaga tgaaagaaag atatggaaca gtctataaag gctctcagag gctcatagac 7560
   gaagaaagtg gagaagtcat agaggtagac aagttatacc gtaaacaaac gtctggtaac   7620
   ttcgtaaagg catatatagt gcaattaata agtatgttag atatgattgg cggaaaaaaa   7680
   cttaaaatcg ttaactatat cctagataat gtccacttaa gtaacaatac aatgatagct   7740
   acaacaagag aaatagcaaa agctacagga acaagtctac aaacagtaat aacaacactt   7800
   aaaatcttag aagaaggaaa tattataaaa agaaaaactg gagtattaat gttaaaccct   7860
   gaactactaa tgagaggcga cgaccaaaaa caaaaatacc tcttactcga atttgggaac   7920
   tttgagcaag aggcaaatga aatagattga cctcccaata acaccacgta gttattggga   7980
   ggtcaatcta tgaaaatgcg atta                                          8004
<210> 16
   <211> 7693
   <212> DNA
   <213> Artificial
<220>
   <223> Plasmid
<400> 16
   agcttggctg caggtcgact tcaagaagag gcacctcatc ttggaaagcc tgtacttgtt     60
   ttaagagatg ttactgaaag accagaagca gttgaggcag gaacggtaaa attagtagga    120
   actgatatta aaaagatagt agacgaagcc tataaaataa tgaaagatga agaagaatat    180
   gaaaaaatga gtaaggcaat aaatccatat ggtgatggaa aggcttcaga tagaatagtt    240
   gatgctatat tgtatcattt tggagtatta aaggatagac cagatgaatt ttcacctaaa    300
   aagtaataca tgagcattct aaaagaattt agaatgaagt ttcttatgca caagtatttt    360
   ttattacatt aatatagtta aaatataaac ttatgtattt atgctaaaac atgattttaa    420
   gggggttagc atatgcataa gtttaatttt tttgttaaaa aatattaaac tttgtgtttt    480
   ttttaacaaa atatattgat aaaaataata atagtgggta taattaagtt gttagagaaa    540
   acgtataaat tagggataaa ctatggaact tatgaaatag attgaaatgg tttatctgtt    600
   accccgtatc aaaatttagg aggttagtta gaatgaaaga agttgtaata gctagtgcag    660
   taagaacagc gattggatct tatggaaagt ctcttaagga tgtaccagca gtagatttag    720
   gagctacagc tataaaggaa gcagttaaaa aagcaggaat aaaaccagag gatgttaatg    780
   aagtcatttt aggaaatgtt cttcaagcag gtttaggaca gaatccagca agacaggcat    840
   cttttaaagc aggattacca gttgaaattc cagctatgac tattaataag gtttgtggtt    900
   caggacttag aacagttagc ttagcagcac aaattataaa agcaggagat gctgacgtaa    960
   taatagcagg tggtatggaa aatatgtcta gagctcctta cttagcgaat aacgctagat   1020
   ggggatatag aatgggaaac gctaaatttg ttgatgaaat gatcactgac ggattgtggg   1080
   atgcatttaa tgattaccac atgggaataa cagcagaaaa catagctgag agatggaaca 1140
   tttcaagaga agaacaagat gagtttgctc ttgcatcaca aaaaaaagct gaagaagcta 1200
   taaaatcagg tcaatttaaa gatgaaatag ttcctgtagt aattaaaggc agaaagggag 1260
   aaactgtagt tgatacagat gagcacccta gatttggatc aactatagaa ggacttgcaa 1320
   aattaaaacc tgccttcaaa aaagatggaa cagttacagc tggtaatgca tcaggattaa 1380
   atgactgtgc agcagtactt gtaatcatga gtgcagaaaa agctaaagag cttggagtaa 1440
   aaccacttgc taagatagtt tcttatggtt cagcaggagt tgacccagca ataatgggat 1500
   atggaccttt ctatgcaaca aaagcagcta ttgaaaaagc aggttggaca gttgatgaat 1560
   tagatttaat agaatcaaat gaagcttttg cagctcaaag tttagcagta gcaaaagatt 1620
   taaaatttga tatgaataaa gtaaatgtaa atggaggagc tattgccctt ggtcatccaa 1680
   ttggagcatc aggtgcaaga atactcgtta ctcttgtaca cgcaatgcaa aaaagagatg 1740
   caaaaaaagg cttagcaact ttatgtatag gtggcggaca aggaacagca atattgctag 1800
   aaaagtgcta gaaaggatcc tgtttaactt taagaaggag atatacatat gttggataat 1860
   ggcttttctt ttcctgttcg tgtgtattat gaagatactg atgcaggtgg cgtagtgtat 1920
   cacgctcgct atttgcattt ttttgaacga gcaagaacag aatatttgcg tacattaaat 1980
   tttacgcaac aaaccttact agaggaacaa caactcgcat ttgttgtcaa aacgctcgcc 2040
   attgattatt gcgtggcagc aaaattggat gatttactta tggtggaaac agaggtttca 2100
   gaagtaaaag gggctacaat cctttttgaa cagagactga tgcgcaacac cctgatgtta 2160
   tcaaaggcta ctgttaaggt agcctgtgtt gatctaggca agatgaaacc agtggcgttt 2220
   cccaaagaag ttaaagcggc gtttcatcac ttaaaataat ttttcggagt atgcaatgag 2280
   gtacctttta tgttaaagga tgaagtaatt aaacaaatta gcacgccatt aacttcgcct 2340
   gcatttccta gaggacccta taaatttcat aatcgtgagt attttaacat tgtatatcgt 2400
   acagatatgg atgcacttcg taaagttgtg ccagagcctt tagaaattga tgagccctta 2460
   gtcaggtttg aaattatggc aatgcatgat acgagtggac ttggttgtta tacagaaagc 2520
   ggacaggcta ttcccgtaag ctttaatgga gttaagggag attatcttca tatgatgtat 2580
   ttagataatg agcctgcaat tgcagtagga agggaattaa gtgcatatcc taaaaagctc 2640
   gggtatccaa agctttttgt ggattcagat actttagtag gaactttaga ctatggaaaa 2700
   cttagagttg cgacagctac aatggggtac aaacataaag ccttagatgc taatgaagca 2760
   aaggatcaaa tttgtcgccc taattatatg ttgaaaataa tacccaatta tgatggaagc 2820
   cctagaatat gtgagcttat aaatgcgaaa atcacagatg ttaccgtaca tgaagcttgg 2880
   acaggaccaa ctcgactgca gttatttgat cacgctatgg cgccacttaa tgatttgcca 2940
   gtaaaagaga ttgtttctag ctctcacatt cttgcagata taatattgcc tagagctgaa 3000
   gttatatatg attatcttaa gtaatagaat tcactggccg tcgttttaca acgtcgtgac 3060
   tgggaaaacc ctggcgttac ccaacttaat cgccttgcag cacatccccc tttcgccagc 3120
   tggcgtaata gcgaagaggc ccgcaccgat cgcccttccc aacagttgcg cagcctgaat 3180
   ggcgaatggc gcctgatgcg gtattttctc cttacgcatc tgtgcggtat ttcacaccgc 3240
   atatggtgca ctctcagtac aatctgctct gatgccgcat agttaagcca gccccgacac 3300
   ccgccaacac ccgctgacgc gccctgacgg gcttgtctgc tcccggcatc cgcttacaga 3360
   caagctgtga ccgtctccgg gagctgcatg tgtcagaggt tttcaccgtc atcaccgaaa 3420
   cgcgcgagac gaaagggcct cgtgatacgc ctatttttat aggttaatgt catgataata 3480
   atggtttctt agacgtcagg tggcactttt cggggaaatg tgcgcggaac ccctatttgt 3540
   ttatttttct aaatacattc aaatatgtat ccgctcatga gacaataacc ctgataaatg 3600
   cttcaataat attgaaaaag gaagagtatg agtattcaac atttccgtgt cgcccttatt 3660
   cccttttttg cggcattttg ccttcctgtt tttgctcacc cagaaacgct ggtgaaagta 3720
   aaagatgctg aagatcagtt gggtgcacga gtgggttaca tcgaactgga tctcaacagc 3780
   ggtaagatcc ttgagagttt tcgccccgaa gaacgttttc caatgatgag cacttttaaa 3840
   gttctgctat gtggcgcggt attatcccgt attgacgccg ggcaagagca actcggtcgc 3900
   cgcatacact attctcagaa tgacttggtt gagtactcac cagtcacaga aaagcatctt 3960
   acggatggca tgacagtaag agaattatgc agtgctgcca taaccatgag tgataacact 4020
   gcggccaact tacttctgac aacgatcgga ggaccgaagg agctaaccgc ttttttgcac 4080
   aacatggggg atcatgtaac tcgccttgat cgttgggaac cggagctgaa tgaagccata 4140
   ccaaacgacg agcgtgacac cacgatgcct gtagcaatgg caacaacgtt gcgcaaacta 4200
   ttaactggcg aactacttac tctagcttcc cggcaacaat taatagactg gatggaggcg 4260
   gataaagttg caggaccact tctgcgctcg gcccttccgg ctggctggtt tattgctgat 4320
   aaatctggag ccggtgagcg tgggtctcgc ggtatcattg cagcactggg gccagatggt 4380
   aagccctccc gtatcgtagt tatctacacg acggggagtc aggcaactat ggatgaacga 4440
   aatagacaga tcgctgagat aggtgcctca ctgattaagc attggtaact gtcagaccaa 4500
   gtttactcat atatacttta gattgattta aaacttcatt tttaatttaa aaggatctag 4560
   gtgaagatcc tttttgataa tctcatgacc aaaatccctt aacgtgagtt ttcgttccac 4620
   tgagcgtcag accccgtaga aaagatcaaa ggatcttctt gagatccttt ttttctgcgc 4680
   gtaatctgct gcttgcaaac aaaaaaacca ccgctaccag cggtggtttg tttgccggat 4740
   caagagctac caactctttt tccgaaggta actggcttca gcagagcgca gataccaaat 4800
   actgtccttc tagtgtagcc gtagttaggc caccacttca agaactctgt agcaccgcct 4860
   acatacctcg ctctgctaat cctgttacca gtggctgctg ccagtggcga taagtcgtgt 4920
   cttaccgggt tggactcaag acgatagtta ccggataagg cgcagcggtc gggctgaacg 4980
   gggggttcgt gcacacagcc cagcttggag cgaacgacct acaccgaact gagataccta 5040
   cagcgtgagc tatgagaaag cgccacgctt cccgaaggga gaaaggcgga caggtatccg 5100
   gtaagcggca gggtcggaac aggagagcgc acgagggagc ttccaggggg aaacgcctgg 5160
   tatctttata gtcctgtcgg gtttcgccac ctctgacttg agcgtcgatt tttgtgatgc 5220
   tcgtcagggg ggcggagcct atggaaaaac gccagcaacg cggccttttt acggttcctg 5280
   gccttttgct ggccttttgc tcacatgttc tttcctgcgt tatcccctga ttctgtggat 5340
   aaccgtatta ccgcctttga gtgagctgat accgctcgcc gcagccgaac gaccgagcgc 5400
   agcgagtcag tgagcgagga agcggaagag cgcccaatac gcaaaccgcc tctccccgcg 5460
   cgttggccga ttcattaatg cagctggcac gacaggtttc ccgactggaa agcgggcagt 5520
   gagcgcaacg caattaatgt gagttagctc actcattagg caccccaggc tttacacttt 5580
   atgcttccgg ctcgtatgtt gtgtggaatt gtgagcggat aacaatttca cacaggaaac 5640
   agctatgacc atgattacgc caagctttgg ctaacacaca cgccattcca accaatagtt 5700
   ttctcggcat aaagccatgc tctgacgctt aaatgcacta atgccttaaa aaaacattaa 5760
   agtctaacac actagactta tttacttcgt aattaagtcg ttaaaccgtg tgctctacga 5820
   ccaaaagtat aaaaccttta agaactttct tttttcttgt aaaaaaagaa actagataaa 5880
   tctctcatat cttttattca ataatcgcat cagattgcag tataaattta acgatcactc 5940
   atcatgttca tatttatcag agctcgtgct ataattatac taattttata aggaggaaaa 6000
   aataaagagg gttataatga acgagaaaaa tataaaacac agtcaaaact ttattacttc 6060
   aaaacataat atagataaaa taatgacaaa tataagatta aatgaacatg ataatatctt 6120
   tgaaatcggc tcaggaaaag ggcattttac ccttgaatta gtacagaggt gtaatttcgt 6180
   aactgccatt gaaatagacc ataaattatg caaaactaca gaaaataaac ttgttgatca 6240
   cgataatttc caagttttaa acaaggatat attgcagttt aaatttccta aaaaccaatc 6300
   ctataaaata tttggtaata taccttataa cataagtacg gatataatac gcaaaattgt 6360
   ttttgatagt atagctgatg agatttattt aatcgtggaa tacgggtttg ctaaaagatt 6420
   attaaataca aaacgctcat tggcattatt tttaatggca gaagttgata tttctatatt 6480
   aagtatggtt ccaagagaat attttcatcc taaacctaaa gtgaatagct cacttatcag 6540
   attaaataga aaaaaatcaa gaatatcaca caaagataaa cagaagtata attatttcgt 6600
   tatgaaatgg gttaacaaag aatacaagaa aatatttaca aaaaatcaat ttaacaattc 6660
   cttaaaacat gcaggaattg acgatttaaa caatattagc tttgaacaat tcttatctct 6720
   tttcaatagc tataaattat ttaataagta agttaaggga tgcataaact gcatccctta 6780
   acttgttttt cgtgtaccta ttttttgtga atcgattatg tcttttgcgc attcacttct   6840
   tttctatata aatatgagcg aagcgaataa gcgtcggaaa agcagcaaaa agtttccttt   6900
   ttgctgttgg agcatggggg ttcagggggt gcagtatctg acgtcaatgc cgagcgaaag   6960
   cgagccgaag ggtagcattt acgttagata accccctgat atgctccgac gctttatata   7020
   gaaaagaaga ttcaactagg taaaatctta atataggttg agatgataag gtttataagg   7080
   aatttgtttg ttctaatttt tcactcattt tgttctaatt tcttttaaca aatgttcttt   7140
   tttttttaga acagttatga tatagttaga atagtttaaa ataaggagtg agaaaaagat   7200
   gaaagaaaga tatggaacag tctataaagg ctctcagagg ctcatagacg aagaaagtgg   7260
   agaagtcata gaggtagaca agttataccg taaacaaacg tctggtaact tcgtaaaggc   7320
   atatatagtg caattaataa gtatgttaga tatgattggc ggaaaaaaac ttaaaatcgt   7380
   taactatatc ctagataatg tccacttaag taacaataca atgatagcta caacaagaga   7440
   aatagcaaaa gctacaggaa caagtctaca aacagtaata acaacactta aaatcttaga   7500
   agaaggaaat attataaaaa gaaaaactgg agtattaatg ttaaaccctg aactactaat   7560
   gagaggcgac gaccaaaaac aaaaatacct cttactcgaa tttgggaact ttgagcaaga   7620
   ggcaaatgaa atagattgac ctcccaataa caccacgtag ttattgggag gtcaatctat   7680
   gaaaatgcga tta                                                      7693

## Beschreibung

### Gebiet der Erfindung

Gegenstand der Erfindung ist ein Verfahren zur Aceton-Produktion.

### Stand der Technik

### ABE-Prozess in Clostridium

Der klassische ABE-Fermentationsprozess, also die mikrobielle Produktion von Aceton, Butanol und Ethanol, war für lange Zeit der weltweit zweitgrößte biotechnologische Prozess direkt nach der Ethanol-Fermentation mit Hefen. Die kommerzielle ABE-Fermentation startete 1916 in England, wo unter anderen Chaim Weizmann die Fähigkeit von *Clostridium acetobutylicum* zur Bildung der Lösungsmittel Aceton, Butanol und Ethanol entdeckte. Der Prozess wurde bis in die späten 50iger Jahre in der westlichen Welt angewendet, in Südafrika sogar noch bis 1981.

Zwei Hauptgründe sind dafür verantwortlich, dass dieser Prozess eingestellt wurde: Zum einen wurde die chemische Synthese von Aceton und Butanol immer günstiger, und zum anderen stieg der Preis für die Substrate der Fermentation stark an. Besonders der Preis für Melasse zog durch deren Verwendung als Futtermittelzusatz für Rinder stark an.

Die steigenden Kosten für petrochemische Vorprodukte, als auch neue technologische Möglichkeiten auf dem Gebiet des Pathway-Engineerings von Mikroorganismen eröffnen nun neue Optionen zur Entwicklung von Hochleistungsstämmen und kommerziellen Fermentationsprozessen zur Produktion von Lösungsmitteln wie Aceton.

Die klassische ABE-Fermentation basiert auf den Organismen *Clostridium acetobutylicum* und *Clostridium beijerinckii.* Beide sind Gram-positiv und vermehren sich unter strikt anaeroben Bedingungen. Diese Organismen können Mono-, Di- und Polysaccharide umsetzen, wobei die hauptsächlich in der Fermentation verwendeten Substrate Melasse und Stärke sind.

Der Fermentationsprozess mit *C. acetobutylicum* unterteilt sich in zwei Phasen. In der ersten Phase geht die Biomassebildung einher mit der Bildung von Acetat, Butyrat und Spuren von Ethanol ("Acidogene Phase"). In der zweiten Phase, der sogenannten "Solventogenen Phase", werden die Säuren dann genutzt, um die Fermentationsprodukte Aceton, Butanol und Ethanol zu bilden (ABE). Die Produkte Aceton, Butanol und Ethanol werden im Wildtyp *C. acetobutylicum* im Verhältnis von ungefähr 3 : 6 : 1 gebildet. Dieses Produktverhältnis kann je nach gewählten Kulturbedingungen (z.B. pH oder Nährstoffzufuhr)oder eingesetzten Substraten stark variieren.

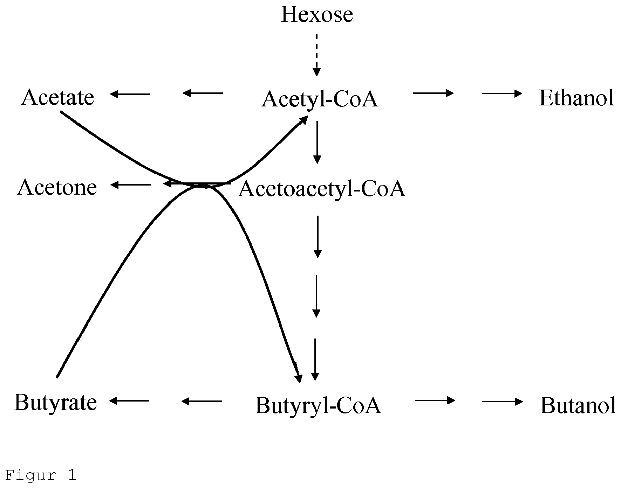

Die Enzyme der Lösungsmittel-Biosynthese von Aceton, Butanol und Ethanol sind weitgehend aufgereinigt und biochemisch charakterisiert (vgl. Figur 1; Duerre, P., und Bahl, H. 1996. Microbial production of acetone/butanol/isopropanol. In: Biotechnology, vol. 6, 2nd ed. M. Roehr, (ed.),VCH Verlagsgesellschaft mbH, Weinheim, Germany. p. 229-268.Duerre, P. 1998. New insights and novel developments in clostridial acetone/butanol/isopropanol fermentation. Appl. Microbiol. Biotechnol.49: 639-648.). Auch die Genomsequenz von C. *acetobutylicum* liegt vor (Noelling, J., Breton, G., Omelchenko, M. V. & 16 other authors(2001). Genome sequence and comparative analysis of the solvent-producing bacterium Clostridium acetobutylicum. J Bacteriol 183,4823-4838.).

Es wurden bereits *Clostridium acetobutylicum-*Stämme generiert, bei denen die Aceton-Produktion von Butanol- und Ethanolproduktion entkoppelt ist, so dass diese Stämme nur Aceton produzieren (Mermelstein et al. (1993). Metabolic engineering of Clostridium acetobutylicum ATCC824 for increased solvent production by enhancement of acetone formation enzyme activities using a synthetic operon. Biotech. Bioeng. 42:1053-1060.; Nair, R.V., and Papoutsakis, E.T. 1994). Die gemessenen Titer für Aceton lagen grundsätzlich unter den Konzentrationen derer im Wildtyp.

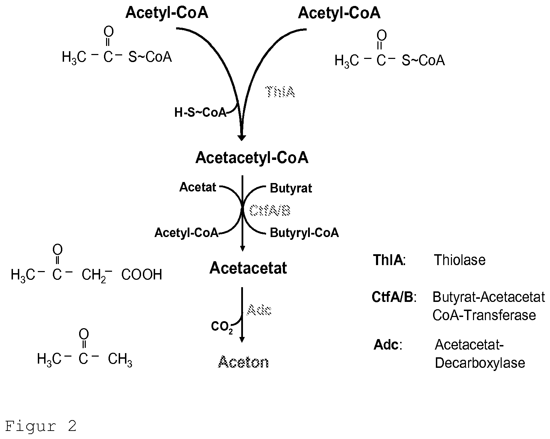

In Figur 2 ist der klassische, in *Clostridium* charakterisierte Stoffwechselweg für die Acetonsynthese dargestellt. Dieser Weg geht aus von Acetyl-CoA, einem zentralen Metaboliten, der in allen Mikroorganismen gebildet wird, unabhängig davon, welche C-Quelle verstoffwechselt wird oder welche Stoffwechselwege etabliert sind. Enzyme, die benötigt werden, sind: die β-Ketothiolase, die zwei Untereinheiten der Acetyl-CoA/Butyryl-CoA-Transferase, und die Acetacetat-Decarboxylase.

Es konnte gezeigt werden, dass die heterologe Expression dieser Enzyme aus *C. acetobutylicum* in *Escherichia coli,* die die Acetonbildung ausgehend von Acetyl-CoA katalysieren (Acetacetat-Decarboxylase, Acetyl-CoA/Butyryl-CoA-Transferase und Thiolase) zu einer Acetonbildung in diesem Organismus von ca. 150 mM führen, wobei aber auch nachteilhafter Weise hohe Mengen an Acetat (50 mM) anfielen (Bermejo L. L., N. E. Welker, E. T. Papoutsakis. 1998. Expression of Clostridium acetobutylicum ATCC 824 Genes in Escherichia coli for Acetone Production and Acetate Detoxification. Appl. Env. Microbiol. 64:1079-1085).

Ein weiterer Nachteil ist hier, dass Aceton nur unter aeroben Bedingungen produziert wurde, da die Redoxequivalente, die während der Metabolisierung von Glukose zu Acetyl-CoA entstehen, unter anaeroben Bedingungen von *E. coli* nicht reoxidiert werden können.

Allen beschriebenen Verfahren ist gemein, dass sie nachteilig komplexe Kohlenstoffquellen wie beispielsweise Zucker benötigen.

### Acetogene Zellen

Acetogene Zellen, daher Zellen, die in der Lage sind, mittels anaerober Atmung Acetat zu bilden, sind bekannt.

Zu den acetogenen Bakterien gehören z.B. Arten der Gattung *Acetobacterium* wie *A. woodii* und *Clostridium aceticum.*

Die WO0068407 beschreibt den Einsatz von acetogenen Bakterien zur Herstellung von Ethanol.

Seit kurzem liegt auch die Genomsequenz von *C. ljungdahlii* vor. Von *C. aceticum* und *C. carboxidivorans* ist noch keine Genomsequenz veröffentlicht. Es ist allerdings bekannt, dass *C. aceticum* zusätzlich ein Plasmid trägt (5,6 kBp, Lee *et al.,* 1987). Derzeit sind keine Techniken publiziert, um diese Organismen genetisch zu verändern.

Die Gruppe der acetogenen Bakterien gehört zu den anaerobe Prokaryoten, die CO2 als terminalen Elektronenakzeptor nutzen können und dabei Acetat bilden. Gegenwärtig werden 21 verschiedenen Gattungen zu den Acetogenen gezählt (Drake *et al*., 2006), wozu auch einige Clostridien zählen (Drake & Küsel, 2005). Sie sind in der Lage, Kohlendioxid plus Wasserstoff oder auch Kohlenmonoxid als Kohlenstoff- und Energiequelle zu nutzen (Wood, 1991). Daneben können auch Alkohole, Aldehyde, Carbonsäuren sowie zahlreiche Hexosen als Kohlenstoff-Quelle genutzt werden (Drake et al., 2004). Der reduktive Stoffwechselweg, der zur Bildung von Acetat führt wird als Acetyl-CoA-oder Wood-Ljungdahl-Weg bezeichnet.

Aufgabe der Erfindung war es, ein Verfahren bereitzustellen, mit welchem Aceton aus ubiquitär verfügbaren Kohlenstoffquellen hergestellt werden kann.

### Beschreibung der Erfindung

Überraschenderweise wurde gefunden, dass das im Folgenden beschriebenen Verfahren die Aufgabe der Erfindung löst.

Gegenstand der vorliegenden Erfindung ist ein Verfahren zur Herstellung von Aceton wie in Anspruch 1 beschrieben.

Ein Vorteil der Erfindung ist, dass die Zellen anaerob und somit energetisch besonders günstig kultiviert werden können.

Ein weiterer Vorteil der Erfindung ist, dass die erfindungsgemäßen Zellen zu einer Verminderung des klimaschädlichen Kohlendioxids beitragen.

Noch ein Vorteil erfindungsgemäßer Zellen ist eine Ausbeutesteigerung durch Acetonproduktion aus Kohlendioxid und Wasserstoff.

Gegenstand der vorliegenden Erfindung ist eine acetogene Zelle, welche Aceton zu bilden vermag.

Unter dem Begriff "acetogene Zelle" im Zusammenhang mit der vorliegenden Erfindung werden Zellen verstanden, die in der Lage sind, mittels anaerober Atmung Acetat zu bilden.

Alle angegebenen Prozent sind, wenn nicht anders angegeben Gewichtsprozent.

Bevorzugt handelt es sich bei der acetogenen Zelle um eine isolierte, insbesondere um eine gentechnisch veränderte Zelle. Erfindungsgemäß bevorzugt ist eine Zelle, die aus mindestens einer Kohlenstoffquelle ausgewählt aus der Gruppe umfassend Kohlendioxid und Kohlenmonoxid Aceton zu bilden vermag. Besonders bevorzugt vermag die erfindungsgemäße acetogene Zelle Aceton aus Kohlenmonoxid und Kohlendioxid als einzige Kohlenstoffquelle zu bilden.

Es ist dem Fachmann hinlänglich bekannt, dass mit Hilfe rekombinanter Gentechnik Produktausbeuten in biologischen Systemen verbessert werden können. Daher ist es erfindungsgemäß weiterhin bevorzugt, dass die acetogene Zelle gegenüber ihrem Wildtyp derart gentechnisch verändert wurde, dass sie im Vergleich zu ihrem Wildtyp mehr Aceton zu bilden vermag.

Die Formulierung "dass sie im Vergleich zu ihrem Wildtyp mehr Aceton zu bilden vermag" betrifft auch den Fall, dass der Wildtyp der gentechnisch veränderten Zelle überhaupt kein Aceton, zumindest aber keine nachweisbaren Mengen dieser Verbindung, zu bilden vermag und erst nach der gentechnischen Veränderung nachweisbare Mengen dieser Komponente gebildet werden können.

Unter einem "Wildtyp" einer Zelle wird vorzugsweise eine Zelle bezeichnet, deren Genom in einem Zustand vorliegt, wie er Gegenstand der vorliegenden Erfindung ist ein Verfahren zur Herstellung von Aceton, umfassend die Verfahrensschritte:
A) in Kontakt Bringen einer acetogenen Zelle, welche Aceton zu bilden vermag, mit einem Nährmedium beinhaltend mindestens eine Kohlenstoffquelle ausgewählt aus der Gruppe umfassend Kohlendioxid und Kohlenmonoxid;
B) Kultivieren der Zelle unter Bedingungen, die es der Zelle ermöglichen, Aceton zu bilden;
C) gegebenenfalls Isolierung des gebildeten Acetons,
dadurch gekennzeichnet,
dass die Zelle eine im Vergleich zu ihrem Wildtyp gesteigerte Aktivität mindestens eines der folgenden Enzyme aufweist:
eines Enzyms E1, welches die Umsetzung von Acetyl-Coenzym A zu Acetacetyl-Coenzym A katalysiert;
eines Enzyms E2, welches die Umsetzung von Acetacetyl-Coenzym A zu Acetacetat katalysiert;
eines Enzyms E3, welches die Umsetzung von Acetacetat zu Aceton katalysiert,
dass die Zelle aus mindestens einer Kohlenstoffquelle ausgewählt aus der Gruppe enthaltend Kohlendioxid und Kohlenmonoxid Aceton zu bilden vermag, und
dass die Zelle gegenüber ihrem Wildtyp derart gentechnisch verändert wurde, dass sie im Vergleich zu ihrem Wildtyp mehr Aceton zu bilden vermag.

Unter dem Begriff "acetogene Zelle" im Zusammenhang mit der vorliegenden Erfindung werden Zellen verstanden, die in der Lage sind, mittels anaerober Atmung Acetat zu bilden. Alle angegebenen Prozent sind, wenn nicht anders angegeben Gewichtsprozent.

Bevorzugt handelt es sich bei der acetogenen Zelle um eine isolierte, insbesondere um eine gentechnisch veränderte Zelle. Erfindungsgemäß ist die Zelle eine, die aus mindestens einer Kohlenstoffquelle ausgewählt aus der Gruppe umfassend Kohlendioxid und Kohlenmonoxid Aceton zu bilden vermag. Besonders bevorzugt vermag die erfindungsgemäße acetogene Zelle Aceton aus Kohlenmonoxid und Kohlendioxid als einzige Kohlenstoffquelle zu bilden.

Es ist dem Fachmann hinlänglich bekannt, dass mit Hilfe rekombinanter Gentechnik Produktausbeuten in biologischen Systemen verbessert werden können die acetogene Zelle wurde gegenüber ihrem Wildtyp derart gentechnisch verändert, dass sie im Vergleich zu ihrem Wildtyp mehr Aceton zu bilden vermag.

Die Formulierung "dass sie im Vergleich zu ihrem Wildtyp mehr Aceton zu bilden vermag" betrifft auch den Fall, dass der Wildtyp der gentechnisch veränderten Zelle überhaupt kein Aceton, zumindest aber keine nachweisbaren Mengen dieser Verbindung, zu bilden vermag und erst nach der gentechnischen Veränderung nachweisbare Mengen dieser Komponente gebildet werden können.

Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Diese Zellen sind gentechnisch derart verändert, dass sie im Vergleich zu ihrem Wildtyp mehr Aceton aus einer Kohlenstoffquelle bilden können.

Dabei ist es erfindungsgemäß bevorzugt, dass die acetogene Zelle derart gentechnisch verändert ist, dass sie in einem definierten Zeitintervall, vorzugsweise innerhalb von 2 Stunden, noch mehr bevorzugt innerhalb von 8 Stunden und am meisten bevorzugt innerhalb von 24 Stunden, mindestens 2mal, besonders bevorzugt mindestens 10mal, darüber hinaus bevorzugt mindestens 100mal, darüber hinaus noch mehr bevorzugt mindestens 1.000mal und am meisten bevorzugt mindestens 10.000mal mehr Aceton bildet als der Wildtyp der Zelle. Die Zunahme der Produktbildung kann dabei beispielsweise dadurch bestimmt werden, dass die erfindungsgemäße Zelle und die Wildtyp-Zelle jeweils getrennt unter gleichen Bedingungen (gleiche Zelldichte, gleiches Nährmedium, gleiche Kulturbedingungen) für ein bestimmtes Zeitintervall in einem geeigneten Nährmedium kultiviert werden und anschließend die Menge an Zielprodukt (Aceton) im Nährmedium bestimmt wird.

Die Zelle weist eine im Vergleich zu ihrem Wildtyp gesteigerte Aktivität mindestens eines der folgenden Enzyme auf:
eines Enzyms E₁, welches die Umsetzung von Acetyl-Coenzym A zu Acetacetyl-Coenzym A katalysiert.
eines Enzyms E₂, welches die Umsetzung von Acetacetyl-Coenzym A zu Acetacetat katalysiert;
eines Enzyms E₃, welches die Umsetzung von Acetacetat zu Aceton katalysiert.

Unter der vorstehend und in den nachfolgenden Ausführungen verwendeten Formulierung "eine im Vergleich zu ihrem Wildtyp gesteigerte Aktivität eines Enzyms Eₓ" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche "eine im Vergleich zu ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms Eₓ induziert wird.

In diesem Zusammenhang besonders bevorzugte Zellen sind dabei diejenigen, in denen die Aktivität des folgenden Enzyms bzw. der folgenden Enzyme erhöht ist: E₁, E₂, E₃, E₁ + E₂ , E₁ + E₃, E₂ + E₃, E₁ + E₂ + E₃, wobei E₁ + E₂ + E₃ besonders bevorzugt ist.

Weiterhin ist es erfindungsgemäß bevorzugt, dass es sich bei dem Enzym E₁ um eine Acetyl-CoA-C-Acetyltransferase (EC 2.3.1.9) handelt;
bei dem Enzym E₂ um eine Butyrat-Acetacetat-CoA-Transferase (EC 2.8.3.9) oder um eine Acyl-CoA-Hydrolase (EC 3.1.2.20) handelt; bei dem Enzym E₃ um eine Acetacetat-Decarboxylase (EC 4.1.1.4) handelt;
Besonders bevorzugt als Enzym E₁ eingesetztes Enzym ist *thlA* aus *Clostridium acetobutylicum.*

Besonders bevorzugt als Enzym E₂ eingesetzte Butyrat-Acetacetat-CoA-Transferase sind *ctfA* und *ctfB* aus *Clostridium acetobutylicum* und *atoD* und *atoA* aus *Escherichia coli.* Besonders bevorzugt als Enzym E₂ eingesetzte Acyl-CoA Hydrolase sind *teII* aus *B. subtilis* bzw. *ybgC* aus *Heamophilus influenzae.* Besonders bevorzugt als Enzym E₃ eingesetztes Enzym ist *adc* aus *Clostridium acetobutylicum.*

Erfindungsgemäße acetogene Zelle ist bevorzugt ein Mikroorganismus, bevorzugt ein Bakterium und besonders bevorzugt ein anaerobes Bakterium, insbesondere ein stabförmiges, grampositives Bakterium.

Ganz besonders bevorzugt werden acetogene Zellen eingesetzt ausgewählt aus der Gruppe umfassend *Thermoanaerobacter kivui, Acetobacterium woodii, Acetoanaerobium notera, Clostridium aceticum, Butyribacterium methylotrophicum, Moorella thermoacetica*, *Eubacterium limosum, Peptostreptococcus productus, Clostridium ljungdahlii* und *Clostridium carboxidivorans.* Ein besonders geeignetes Bakterium ist *Clostridium carboxidivorans,* insbesondere solche Stämme wie "P7" und "P11". Solche Zellen sind beispielsweise in US 2007/0275447 und US 2008/0057554 beschrieben.

Ein weiteres, besonders geeignetes Bakterium ist *Clostridium ljungdahlii,* insbesondere Stämme ausgewählt aus der Gruppe umfassend *Clostridium ljungdahlii* PETC, *Clostridium ljungdahlii* ERI2, *Clostridium ljungdahlii* C01 und *Clostridium ljungdahlii* O-52 und werden in der WO 98/00558 und WO 00/68407 beschrieben.

Die erfindungsgemäße acetogene Zelle vermag, bevorzugt unter anaeroben Bedingungen, Aceton aus mindestens einer Kohlenstoffquelle ausgewählt aus der Gruppe enthaltend Kohlendioxid und Kohlenmonoxid zu bilden.

In Bezug auf die Quelle dieser Substrate ist offenbar, dass viele mögliche Quellen zur Bereitstellung von CO bzw. CO₂ als Kohlenstoffquelle existieren. Es ist ersichtlich, dass in der Praxis als Kohlenstoffquelle der vorliegenden Erfindung jedes Gas bzw. jede Gasmischung eingesetzt werden kann, welche in der Lage ist, die acetogene Zelle mit genügenden Mengen an Kohlenstoff zu versorgen, damit diese ihre anaerobe Amtung durchführen und Aceton zu bilden vermag.

In dem erfindungsgemäßen Verfahren ist es bevorzugt, dass die Kohlestoffquelle bereitgestellt wird durch Abgase wie beispielsweise Synthesegas, Rauchgas, Erdölraffinerieabgase, durch Hefegärung oder clostridielle Gärung entstandene Gase, Abgase aus der Vergasung zellulosehaltige Materialien oder der Kohlevergasung.

Diese Abgase müssen nicht notwendigerweise als Nebenerscheinungen anderer Prozesse entstanden sein, sondern können extra für den Einsatz in dem erfindungsgemäßen Verfahren produziert werden.

Es ist ersichtlich, dass in der Praxis als Kohlenstoffquelle der vorliegenden Erfindung jedes Abgas eingesetzt werden kann, welches in der Lage ist, die acetogene Zelle mit genügenden Mengen an Kohlenstoff zu versorgen, damit diese ihre anaerobe Atmung durchführen kann.

In einer bevorzugten Ausgestaltung des erfindungsgemäßen Verfahrens ist die Kohlenstoffquelle Synthesegas.

Synthesegas kann z.B. aus dem Nebenprodukt der Kohlevergasung bereitgestellt werden. Die acetogene Zelle wandelt folglich eine Substanz, die ein Abfallprodukt ist, in einen wertvollen Rohstoff um.

Alternativ kann Synthesegas durch die Vergasung von weit verfügbaren, preiswerten landwirtschaftlichen Rohstoffen für das erfindungsgemäße Verfahren bereitgestellt werden.

Es gibt zahlreiche Beispiele an Rohstoffen, die in Synthesegas umgewandelt werden können, da fast alle Vegetationsformen zu diesem Zwecke genutzt werden können. Bevorzugte Rohstoffe sind ausgewählt aus der Gruppe umfassend perennierende Gräser wie Chinaschilf, Getreiderückstände, Verarbeitungsabfälle wie Sägemehl.

Im Allgemeinen wird Synthesegas in einer Vergasungsapparatur aus getrockneter Biomasse gewonnen, hauptsächlich durch Pyrolyse, partielle Oxidation und Dampfreformierung, wobei die Primärprodukte CO, H₂ und CO₂ sind.

Normalerweise wird ein Teil des Produktgases aufbereitet, um Produkterträge zu optimieren und Teerbildung zu vermeiden. Das Cracken des unerwünschten Teers in Synthesegas und CO kann unter Einsatz von Kalk und/oder Dolomit durchgeführt werden. Diese Prozesse werden im Detail in z.B. Reed, 1981 beschrieben (Reed, T.B.,1981, Biomass gasification: principles and technology, Noves Data Corporation, Park Ridge, NJ.)

Es können auch Mischungen verschiedener Quellen als Kohlenstoffquelle eingesetzt werden.

Die im erfindungsgemäßen Verfahren eingesetzten Nährmedien müssen in geeigneter Weise den Ansprüchen der jeweiligen Stämme genügen. Beschreibungen von Nährmedien verschiedener Mikroorganismen sind im Handbuch "Manual of Methods for General Bacteriology" der American Society for Bacteriology (Washington D. C., USA, 1981) enthalten.

Neben den Kohlenstoffquellen enthält das Nährmedium insbesondere Stickstoff und Phosphorquellen, Salze sowie Mittel zur pH-Kontrolle.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze im Nährmedium enthalten sein. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden.

Im Verfahrensschritt B) des erfindungsgemäßen Verfahrens werden die acetogenen Zellen unter Bedingungen, die es der Zelle ermöglichen, Aceton zu bilden, kultiviert. Bevorzugt findet diese Kultivierung unter anaeroben Bedingungen statt.

Die erfindungsgemäßen, gentechnisch veränderten Zellen können dabei kontinuierlich oder diskontinuierlich im *batch*-Verfahren (Satzkultivierung) oder im *fed-batch*-Verfahren (Zulaufverfahren) oder *repeated-fed-batch*-Verfahren (repetitives Zulaufverfahren) zum Zwecke der Produktion von Aceton mit dem Nährmedium in Kontakt und somit kultiviert werden.

Denkbar ist auch ein semi-kontinuierliches Verfahren, wie es in der GB-A-1009370 beschrieben wird. Eine Zusammenfassung über bekannte Kultivierungsmethoden ist im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.

Weitere gut geeignete Verfahren zu Kultivierung der acetogenen Zelle in Verfahrensschritt b) sind z.B. in dem DOE report "Bench-scale Demonstration of Biological Production of Ethanol from Coal Synthesis Gas", Topical Report 5, November 1995 (DOE Contract Number DE-AC22-92PC92118) und in den Schriften WO 98/00558, WO 00/68407 und WO 02/08438 beschrieben.

Im Schritt C) des erfindungsgemäßen Verfahrens kann das durch die Zellen gebildete Aceton gegebenenfalls aus den Zellen und/oder dem Nährmedium isoliert werden, wobei zur Isolierung alle dem Fachmann bekannten Methoden zur Isolierung von niedermolekularen Substanzen aus komplexen Zusammensetzungen in Betracht kommen.

Beispielhaft seien an dieser Stelle die Fällung mittels geeigneter Lösungsmittel, die Extraktion mittels geeigneter Lösungsmittel, die Komplexierung, beispielsweise mittels Cyclodextrinen oder Cyclodextrin-Derivaten, die Kristallisation, die Aufreinigung bzw. Isolierung mittels chromatographischer Methoden oder die Überführung des Acetons in leicht abtrennbare Derivate genannt.

Insbesondere destillative Trennverfahren sind für den Einsatz in Verfahrensschritt C) geeignet.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Figuren sind Bestandteil der Offenbarung:
Figur 1: Biosyntheseweg des klassischen, clostridiellen ABE-Prozesses
Figur 2: Biosyntheseweg von Aceton in *C*. *acetobutylicum*
Figur 3: Plasmidkarte pUC_adc_ctfAB_thlA:
Figur 4: Plasmidkarte pIMP_adc_ctfAB_thlA:

### Beispiele:

### Beispiel 1. Klonierung der Expressionsvektoren

Für die Acetonproduktion in acetogenen Zellen wurden die Klonierungen in *E. coli* XL2 blue durchgeführt. Hierfür wurden die Gene
*ctfA und ctfB* aus *Clostridium acetobutylicum,*
*bzw. atoA* und *atoD* aus *E. coli,*
bzw. *teII* aus *B. subtilis,*
so wie bzw. *ybgC* aus *Heamophilus influenzae*
zusammen mit den Genen *thlA* und *adc* aus *C*. *acetobutylicum* auf dem Plasmid pIMP1 (Mermelstein et al., 1992) arrangiert.

Eine Übersicht über die korrespondierenden Expressionsplasmide ist in Tabelle 1 zusammengestellt.

**Tabelle 1: Plasmide**

| **Plasmid** | **CoA-Transferase/ Thioesterase aus** | **Seq ID No** |
|---|---|---|
| pIMP_adc_ctfAB_thlA | *C. acetobutylicum* | *13* |
| pIMP_adc_atoDA_thlA | *E. coli* | *14* |
| pIMP_adc_teII_thlA | *B. subtilis* | *15* |
| pIMP_adc_ybgC_thlA | *H. influenzae* | *16* |

Die Klonierung der Gene erfolgte sequentiell. Hierzu wurden zunächst Oligonukleotide (Tabelle 2) für die Amplifikation der Gene unter Einführung entsprechender Schnittstellen entworfen und anschließend alle Fragmente amplifiziert.

**Tabelle 1: Oligonukleotide**

| **Name** | **Sequenz (5' → 3')*)** | **Schnittstelle** | **Seq ID No** |
|---|---|---|---|
| ***adc* fw** | GGAA**GGTA**CCTTTTATG | *Acc*65I | *1* |
| ***adc* rev** | GTAACTCT**GAATTC**TATTACTTAAG | *Eco*RI | *2* |
| ***atoDA* fw** | CACAACGGT**GGATC**CAAGAG | *Bam*HI | *3* |
| ***atoDA* rev** | CGCGATAT**GGTACC**AATCAT | *Acc*65I | *4* |
| ***ctfAB* fw** | GAATTTAAAAGGAG**GGATCC**AAATGAAC | *Bam*HI | *5* |
| ***ctfAB* rev** | GTTTCATAGTATT**GGTACC**TAAACAGC | *Acc*65I | *6* |
| ***thl* fw** | CTCAG**GTCGAC**TTCAAGAAG | *Sal*I | *7* |
| ***thl* rev** | CAGAGTTATTTTTAA**GGATCC**TTTCTAGC | *Bam*HI | *8* |
| ***telI fw*** | CAATTG**GGATCC**GATAACAATTTCACACAG | *Bam*HI | *9* |
| ***telI rev*** | GAGATCT**GGTACC**CGGTTAAATGATCGGA | *Acc*65I | *10* |
| ***ybgc fw*** | CTCTAGAA**GGATCC**TGTTTAACTTTAAG | *Bam*HI | *11* |
| ***ybgc rev*** | ATTGG**GTACC**TCATTGCATACTCCG | *Acc*65I | *12* |

Korrespondierende Fragmente wurden von genomischer DNA mittels herkömmlicher PCR-Methoden amplifiziert, elektrophoretisch getrennt und aufgereinigt.

Generierung pUC_adc_ctfAB_thlA:
Im ersten Schritt wurde *adc* über die Schnittstellen *Acc*65I und *Eco*RI in den Vektor pUC18 kloniert und anschließend *thlA* über *Sal*I und *Bam*HI zugefügt. Im letzten Schritt wurden *ctfA* und *ctfB* in einem Schritt, da es als Operon in *C*. *acetobutylicum* organisiert ist, über die Schnittstellen *Bam*HI und *Acc*65I kloniert. Im daraus resultierten Vektor pUC_adc_ctfAB_thlA liegen nun die für die Acetonproduktion nötigen Gene in einem Operon organisiert vor.

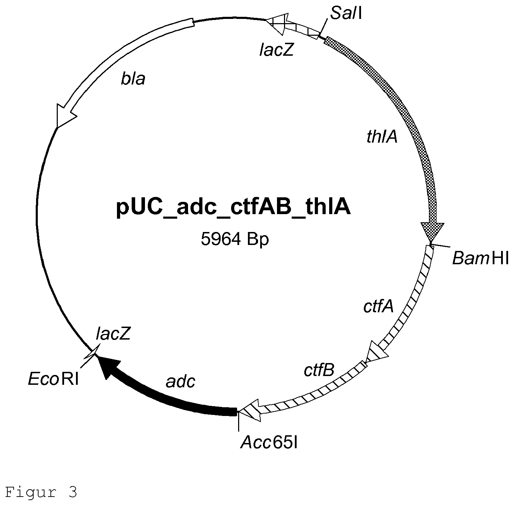

Figur 3 zeigt das resultierende pUC-Plasmid.

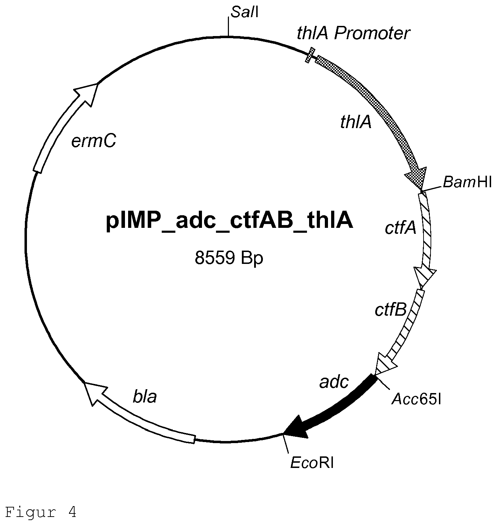

Generierung pIMP_adc_ctfAB_thlA:
Anschließend erfolgte die Umklonierung der Genkassette in den Vektor pIMP1 über die Restriktionsendonukleasen SalI und EcoRI, woraus das Expressionsplasmid pIMP_adc_ctfAB_thlA resultierte, vgl Figur 4.

Generierung pIMP_adc_atoDA_thlA, pIMP_adc_teII_thlA und pIMP_adc_ybgC_thlA:
Zur Generierung der Vektoren pIMP_adc_atoDA_thlA, pIMP_adc_teII_thlA und pIMP_adc_ybgC_thlA wurden die Gene *ctfA* und *ctfB* aus dem Vektor pIMP_adc_ctfAB_thlA über die Schnittstellen *Bam*HI und *Acc*65I ausgeschnitten. *atoD* und *ato*A, welche in *E. coli* in einem Operon organisiert sind, wurden amplifiziert, und ebenso die Gene *teII* aus *B. subtilis* und *ybgC* aus *H. infuenzae,* wobei die Schnittstellen *Bam*HI und *Acc*65I generiert wurden.

Diese Fragmente wurden zuerst über diese in den Vektor pDrive (*atoDA*) bzw. pUC19 (*teII* und *ybgC*) kloniert. Anschließend erfolgte die Umklonierung dieser Gene aus dem pDrive- bzw. pUC-Vektoren, wofür die Genkassetten über die Schnittstellen *Bam*HI und *Acc*65I geschnitten, aufgereinigt und in das wie oben beschrieben restringierte (*Acc*65I und *BamHI*) und aufgereinigte Vektorrückgrat des pIMP_adc_ctfAB_thlA ligiert wurden.

### Beispiel 2: Acetonsynthese in E. coli

Alle erhaltenen Plasmid-Varianten (siehe Tabelle 1) wurden zur Kontrolle auf Funktionalität im *E*. *coli*-Klonierstamm XL2-blue auf Acetonbildung hin untersucht. Die Analysen erfolgten im 100-ml-Maßstab in TY-Medium mit Ampicillin (100 µg/ml). Nach Inokulation aus entsprechenden Vorkulturen auf eine optische Dichte (600 nm) von 0,1 erfolgte eine Inkubation bei 37 °C und 150 Upm. Die optische Dichte wurde photometrisch verfolgt und zu bestimmten Zeitpunkten wurden über einen Zeitraum von ca. 50 h Proben entnommen und gaschromatographisch die Konzentration von Aceton und Acetat im zellfreien Medienüberstand bestimmt. Hierbei zeigte sich, dass mit der Kombination clostridieller Gene (*thlA* und *adc*) mit *atoDA* aus *E. coli* bis zu 80 mM Aceton produziert wurden. Mit rein clostridiellen Genen (*thlA, ctfAB, adc*) wurden 5 mM Aceton produziert und mit den Kombinationen clostridielle Gene (*thlA* und *adc*) mit *teII* aus *B. subtilis* bzw. *ybgC aus H. influenzae* 1 mM Aceton.

### Beispiel 3: Acetogene Acetonproduktion

Je nach eingesetztem Clostridienstamm wurden verschiedene Medien eingesetzt:
Zur Herstellung der Medien für *C*. *carboxidivorans* bzw. *C*. *ljungdhalii* wurden die Chemikalien eingewogen, in Wasser gelöst und anschließend der pH-Wert eingestellt. Zusätzlich wurde der Redox-Indikator Reszurin (1 mg/l) zugesetzt, um das Redoxpotential und somit den Sauerstoffgehalt später prüfen zu können. Anschliessend wurden die Medien in einem Heizpilz aufgekocht und im Eisbad abgekühlt. Während dessen erfolgte die Begasung mit Stickstoff, um den gelösten Sauerstoff zu entfernen. Anschliessend wurden die Medien in die Anaerobenkammer eingeschleust, das Endvolumen mit anaerobem Wasser eingestellt, abgefüllt und luftdicht verschlossen. Wenn eine andere Gasphase als Stickstoff verwendet werden sollte, erfolgte im Anschluss ein Gasaustausch, wobei das Medium mit dem entsprechenden Gas mittels einer langen Kanüle begast wurde und letztendlich ein leichter Überdruck von rund 0,8 bar angelegt wurde.

Für das Medium für *C*. *aceticum* wurden alle Komponenten eingewogen, gelöst und abgefüllt. Zusätzlich wurde der Redox-Indikator Reszurin (1 mg/l) zugesetzt, um das Redoxpotential und somit den Sauerstoffgehalt später prüfen zu können. Anschliessend erfolgte die Begasung über Kanülen mit einer Mischung aus 80 % N₂ und 20 % CO₂, bis ein pH von 7,4 erreicht wurde. Auch hier wurde ein leichter Überdruck angelegt. Nach dem Autoklavieren wurde steril Na₂CO₃ in Form einer anaeroben 5 %igen Na₂CO₃-Lösung zugegeben, um einen pH von 8,2 zu erreichen. Zusätzlich wurde Fructose steril zu einer Endkonzentration von 1 % zugegeben. Für autotrophes Wachstum wurde eine Gasatmosphäre von 80 % H₂ und 20 % CO₂ erzeugt.

Alle Medien wurden bei 121 °C und 1,2 bar für 15 min autoklaviert. Einige Medienbestandteile wurden separat autoklaviert, um chemische Reaktionen der Komponenten untereinander zu verhindern. Hitzelabile Komponenten wurden gelöst, sterilfiltriert und dem abgekühlten, autoklavierten Medium vor Benutzung zugesetzt.

Für die Herstellung von festen Medien wurden 1,5 % (w/v) Agar vor dem Autoklavieren zugegeben und direkt danach wurden in der Anaerobenkammer diese in Petrischalen gegossen. Nach dem Gießen wurden die Platten einige Tage lang getrocknet und bis zur Verwendung bei 4 °C gelagert.

Medium für *C*. *aceticum*

| | | |
|---|---|---|
| NH₄Cl | 1,00 g | 18,7 mM |
| KH₂PO₄ | 0,33 g | 2,4 mM |
| K₂HPO₄ | 0,45 g | 2,6 mM |
| MgSO₄ x 7 H₂O | 0,10 g | 0,4 mM |
| Spurenelementelösung (s.u.) | 20, 00 ml | 2 % (v/v) |
| Wolfe's Vitaminlösung (s.u.) | 20, 00 ml | 2 % (v/v) |
| Hefeextract | 2,00 g | 0,2 % (w/v) |
| NaHCO₃ | 10,00 g | 0,1 M |
| Cystein-HCl x H₂O | 0,50 g | 2,8 mM |
| Na₂S x 9 H₂O | 0,50 g | 2,1 mM |
| Wasser | ad 1000 ml | |

Nach dem Autoklavieren wurde steril 25 ml 1-1 einer 5 Gew.-%igen Na₂CO₃-Lösung zugegeben, um einen pH von 8,2 zu erreichen. Zusätzlich wurde Fructose steril zu einer Endkonzentration von 1 Gew.-% bezogen auf das gesamte Medium zugegeben. Für autotrophes Wachstum wurde vor dem autoklavieren eine Gasatmosphäre von 80 Vol.-% H₂ und 20 Vol.-% CO₂ erzeugt.

Medium für *C*. *carboxidivorans -* Wilkins-Chalgren Medium

| | | |
|---|---|---|
| Wilkins-Chalgren Anaerobe Broth (OXOID CM0643) | 33 g | 3,3 % |
| NaHCO₃ | 1 g | 12 mM |
| Wasser | ad 1000 ml | |

Der pH wurde vor dem Aufkochen und Anaerobisieren auf 5,6 einstellt und nach dem Autoklavieren wurden 10 ml Reduktionsmittel 1 (siehe unten) zugeben, wonach der pH bei 6,0 liegen sollte.

Medium für *C*. *ljungdahlii -* ATCC Medium 1754 (PETC Medium)

| | | |
|---|---|---|
| NH₄Cl | 1,0 g | 19 mM |
| KCl | 0,1 g | 1,35 mM |
| MgSO₄ * 7 H₂O | 0,2 g | 0,8 mM |
| NaCl | 0,8 g | 14 mM |
| KH₂PO₄ | 0,1 g | 0,7 mM |
| CaCl₂ * 2 H₂O | 20,0 mg | 0,15 mM |
| Hefeextrakt | 1,0 g | 0,1 % (w/v) |
| Spurenelementlösung | 10,0 ml | 1 % (v/v) |
| Wolfe's Vitaminlösung | 10,0 ml | 1 % (v/v) |
| NaHCO₃ | 2,0 g | 24 mM |
| Wasser | ad 1000 ml | pH 5,5 |

Vor dem Aufkochen und Anaerobisieren wurde der pH auf 5,5 einstellt. Nach dem Autoklavieren wurden 20 ml einer sterilen Fructoselösung (250 g/l) und je 5 ml Reduktionsmittel 1 und 2 (siehe unten) zugeben, der pH sollte danach bei 5,9 liegen.

### Reduktionsmittel 1

Es werden 1,8 g NaOH in 200 ml Wasser gelöst, aufgekocht und unter Stickstoffbegasung abgekühlt. In der Anaerobenkammer werden in 100 ml anaerobem NaOH zuerst 4 g L-Cystein-HCl und anschließend 4 g Na₂S * 9 H₂O gelöst und anschließend autoklaviert.

### Reduktionsmittel 2

Es werden 1,8 g NaOH in 200 ml Wasser gelöst, aufgekocht und unter Stickstoffbegasung abgekühlt. In der Anaerobenkammer werden in 100 ml anaerobem NaOH 4 g L-Cystein-HCl gelöst und anschließend autoklaviert.

### Spurenelementlösung für das ATCC Medium 1754 und für das C. aceticum-Medium

| | | |
|---|---|---|
| Nitrilotriessigsäure | 2 g | 10,5 mM |
| MnSO₄ * H₂O | 1 g | 6 mM |
| Fe (SO₄)₂(NH4 )₂ * 6 H₂O | 0,8 g | 2 mM |
| CoCl₂ * 6 H₂O | 0,2 g | 0, 86 mM |
| ZnSO₄ * 7 H₂O | 0,2 mg | 0,7 µm |
| CuCl₂ * 2 H₂O | 20 mg | 0,12 mM |
| NiCl₂ * 6 H₂O | 20 mg | 80 µM |
| Na₂MoO₄ * 2 H₂O | 20 mg | 80 µM |
| Na2SeO4 | 20 mg | 80 µM |
| Na₂WO₄ | 20 mg | 60 µM |
| Wasser | ad 1000 ml | |

Zuerst wurde die Nitrilotriessigsäure vollständig in Wasser gelöst, der pH-Wert mit Kaliumhydroxid auf 6,0 eingestellt und danach die anderen Komponenten gelöst.

Wolfe's Vitaminlösung für das ATCC Medium 1754 und für das *C. aceticum*-Medium

| | | |
|---|---|---|
| Biotin (Vitamin H) | 2,0 mg | 8 µM |
| Folsäure (Vitamin B9) | 2,0 mg | 4,5 µM |
| Pyridoxin-HCl (Vitamin B6) | 10,0 mg | 49 µM |
| Thiamin-HCl (Vitamin B1) | 5,0 mg | 15 µM |
| Riboflavin (Vitamin B2) | 5,0 mg | 13 µM |
| Nicotinsäureamid (Vitamin PP) | 5,0 mg | 41 µM |
| Calcium D-(+)-Pantothenat | 5,0 mg | 10,5 µM |
| Cyanocobalmin (Vitamin B12) | 0,1 mg | 74 µM |
| p-Aminobenzoesäure | 5,0 mg | 36 µM |
| Liponsäure | 5,0 mg | 24 µM |
| Wasser | ad 1000 ml | pH 4,3 |

Die in *E. coli* X12-blue konstruierten Plasmide wurden anschließend in acetogene Clostridien mittels Konjugation (Purdy et al., 2002) bzw. Transformation (Zhu *et al.*, 2004) eingebracht, wodurch der rekombinante Stamm die Fähigkeit erhält, Aceton zu produzieren.

Für die Konjugationsexperimente wurde der *E*. *coli*-Donorstamm CA434 mit dem zu übertragenden Plasmid in LB-Medium aerob über Nacht angezogen. Ein 1-ml-Aliquot wurde 1 min bei 10000 × g zentrifugiert und das Zellsediment in der Anaerobenkammer in 1 ml sterilem, anaeroben PBS-Puffer (1,5 mM KH₂PO₄, 4,2 mM Na₂HPO₄, 137 mM NaCl, 2,7 mM KCl) vorsichtig suspendiert, um ein Abscheren der konjugativen Pili zu verhindern. Die Zellen wurden erneut zentrifugiert und in 200 µl einer über Nacht in entsprechendem Medium gewachsenen Clostridienkultur aufgenommen. In der Anaerobenkammer wurde dieses Gemisch auf gut getrocknete Agarplatten in 10-µl-Tropfen verteilt und bei 37 °C für 6 h anaerob inkubiert. Anschließend wurden die Zellen 2- bis 3mal mit jeweils 0,5 ml sterilem, anaerobem PBS-Puffer von der Agarplatte abgewaschen. Das Konjugationsgemisch wurde auf Selektiv-Agarplatten (Clarithromycin) ausplattiert und anaerob bei 37 °C inkubiert.

Für die Transformation wurden die clostridiellen Zellen in 50 ml C. aceticum-Medium mit 40 mM DL-Threonin bei 30 °C bis zu einer optische Dichte von 0,3 - 0,4 angezogen. Die folgenden Schritte erfolgten in der Anaerobenkammer. Hier wurden die Zellen geerntet (6000 Upm, 10 min, RT), zweimal mit SMP-Puffer (270 mM Saccharose, 1 mM MgCl₂, 7 mM NaH₂PO₄) gewaschen und letztendlich in 500 bis 700 µl SMP-Puffer aufgenommen und diese in die Tranformation eingesetzt. Hierfür wurden die Zellen in Elektroporationsküvetten (4 mm) transferiert und mit 0,5 bis 1,5 µg Plasmid-DNA versetzt. Nach 5-minütiger Inkubation erfolgte die Elektroporation bei 25 µF, 600 Ω und 2,5 kV in einem Gene-Pulser (Bio-Rad Laboratories GmbH; München) mit 4 mM Küvetten (Biozym Scientific GmbH). Anschließend wurden die Zellen sofort in 5 ml vorgewärmtes Medium gegeben. Es folgte eine Inkubation zur Resistenzausprägung bei 37 °C über Nacht bis hin zu vier Tagen, woraufhin 5 ml Medium mit Clarithromycin (5 µg ml⁻¹) inokuliert und für 3 bis 5 Tagen bei 37 °C inkubiert wurden.

Um die Transformation zu überprüfen, folgte eine Plasmidisolierung mittels "peqGOLD^{®} Plasmid Miniprep Kit II" (Peqlab, Erlangen). Die Präparation erfolgte nach den Angaben des Herstellers, wobei alle optionalen Schritte durchgeführt wurden. Anschließend erfolgte eine "Plasmidrecovery", wobei der *E. coli*-Stamm XL2-blue verwendet wurde und darauf folgend ein Restriktionsverdau.

Alle erhaltenen Plasmid-Varianten (siehe Tabelle 1) wurden in den autotrophen Clostridien auf Acetonbildung hin untersucht. Die Analysen erfolgten im 50-ml-Maßstab im entsprechenden Medium mit Clarithromycin (5 µg/ml). Nach Inokulation aus entsprechenden Vorkulturen erfolgte eine Inkubation bei 37 °C. Die notwendige Begasung des Mediums erfolgte bei der Medienherstellung. Zum Einsatz kamen hier entweder Synthesegas oder ein CO₂-/H₂-Gemisch im Verhältnis 1:2. Die optische Dichte wurde photometrisch verfolgt und zu bestimmten Zeitpunkten wurden über einen Zeitraum von ca. 100 bis 200 h Proben entnommen und gaschromatographisch die Konzentration von Aceton und Acetat im zellfreien Medienüberstand bestimmt. Hierbei zeigt sich, dass mit der Kombination clostridielle Gene (*thlA* und *adc*) mit *atoDA* aus *E. coli* und mit den Kombinationen clostridielle Gene (*thlA* und *adc*) mit *teII* aus *B. subtilis* bzw. *ybgC aus H. influenzae* bis zu 1 mM Aceton produziert wird. Mit rein clostridiellen Genen (*thlA, ctfAB, adc*) wurde bis zu 0,24 mM Aceton produziert.

## Patentansprüche

1. Ein Verfahren zur Herstellung von Aceton, umfassend die Verfahrensschritte:
A) in Kontakt Bringen einer acetogenen Zelle, welche Aceton zu bilden vermag, mit einem Nährmedium beinhaltend mindestens eine Kohlenstoffquelle ausgewählt aus der Gruppe umfassend Kohlendioxid und Kohlenmonoxid;
B) Kultivieren der Zelle unter Bedingungen, die es der Zelle ermöglichen, Aceton zu bilden;
C) gegebenenfalls Isolierung des gebildeten Acetons,
**dadurch gekennzeichnet,**
**dass** die Zelle eine im Vergleich zu ihrem Wildtyp gesteigerte Aktivität mindestens eines der folgenden Enzyme durch Überexpression aufweist:
eines Enzyms E₁, welches die Umsetzung von Acetyl-Coenzym A zu Acetacetyl-Coenzym A katalysiert;
eines Enzyms E₂, welches die Umsetzung von Acetacetyl-Coenzym A zu Acetacetat katalysiert;
eines Enzyms E₃, welches die Umsetzung von Acetacetat zu Aceton katalysiert, und dass die Zelle aus mindestens einer Kohlenstoffquelle ausgewählt aus der Gruppe enthaltend Kohlendioxid und Kohlenmonoxid Aceton zu bilden vermag.

2. Verfahren gemäß Anspruch 1, **dadurch gekennzeichnet, dass** es sich
bei dem Enzym E₁ um eine Acetyl-CoA-C-Acetyltransferase (EC 2.3.1.9) handelt;
bei dem Enzym E₂ um eine Butyrat-Acetacetat-CoA-Transferase (EC 2.8.3.9) oder um eine Acyl-CoA-Hydrolase (EC 3.1.2.20) handelt;
bei dem Enzym E₃ um eine Acetacetat-Decarboxylase (EC 4.1.1.4) handelt.

3. Verfahren gemäß mindestens einem der Ansprüche 1 oder 2, **dadurch gekennzeichnet, dass** die Zelle ein Mikroorganismus ist, ausgewählt aus der Gruppe umfassend *Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii* und *Clostridium carboxidivorans.*

## Claims

1. A method for producing acetone, comprising the process steps:
A) contacting an acetogenic cell that is able to form acetone with a nutrient medium comprising at least one carbon source selected from the group comprising carbon dioxide and carbon monoxide;
B) cultivating the cell under conditions that enable the cell to form acetone;
C) optionally isolating the acetone that has formed,
**characterized in that**, compared with its wild type, the cell has increased activity of at least one of the following enzymes by overexpression:
an enzyme E₁, which catalyses the reaction of acetyl-coenzyme A to acetoacetyl-coenzyme A;
an enzyme E₂, which catalyses the reaction of acetoacetyl-coenzyme A to acetoacetate;
an enzyme E₃, which catalyses the reaction of acetoacetate to acetone,
and **in that** the cell is able to form acetone from at least one carbon source selected from the group comprising carbon dioxide and carbon monoxide.

2. Method according to Claim 1, **characterized in that** the enzyme E₁ is an acetyl-CoA-C-acetyl transferase (EC 2.3.1.9);
the enzyme E₂ is a butyrate-acetoacetate-CoA-transferase (EC 2.8.3.9) or an acyl-CoA-hydrolase (EC 3.1.2.20);
the enzyme E₃ is an acetoacetate decarboxylase (EC 4.1.1.4).

3. Method according to at least one of Claims 1 and 2, **characterized in that** the cell is a microorganism, selected from the group comprising *Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii* and *Clostridium carboxidivorans.*

## Revendications

1. Procédé de fabrication d'acétone, comprenant les étapes de procédé suivantes :
A) la mise en contact d'une cellule acétogène, capable de former de l'acétone, avec un milieu nutritif contenant au moins une source de carbone choisie dans le groupe comprenant le dioxyde de carbone et le monoxyde de carbone ;
B) la culture de la cellule dans des conditions qui permettent à la cellule de former de l'acétone ;
C) éventuellement l'isolement de l'acétone formée,
**caractérisé en ce que**
la cellule présente une activité augmentée par rapport à son type sauvage d'au moins une des enzymes suivantes par surexpression :
une enzyme E₁, qui catalyse la réaction d'acétyl-coenzyme A en acétacétyl-coenzyme A ;
une enzyme E₂, qui catalyse la réaction d'acétacétyl-coenzyme A en acétacétate ;
une enzyme E₃, qui catalyse la réaction d'acétacétate en acétone,
et
**en ce que** la cellule est capable de former de l'acétone à partir d'au moins une source de carbone choisie dans le groupe contenant le dioxyde de carbone et le monoxyde de carbone.

2. Procédé selon la revendication 1, **caractérisé en ce que**
l'enzyme E₁ est une acétyl-CoA-C-acétyltransférase (EC 2.3.1.9) ;
l'enzyme E₂ est une butyrate-acétacétate-CoA-transférase (EC 2.8.3.9) ou une acyl-CoA-hydrolase (EC 3.1.2.20) ; l'enzyme E₃ est une acétacétate-décarboxylate (EC 4.1.1.4).

3. Procédé selon au moins l'une quelconque des revendications 1 ou 2, **caractérisé en ce que** la cellule est un microorganisme choisi dans le groupe comprenant *Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii* et *Clostridium carboxidivorans.*
